Europäisches Patentamt

European Patent Office

Office européen des brevets

(19)

(11) **EP 0 771 325 B1**

(12) **EUROPÄISCHE PATENTSCHRIFT**

(45) Veröffentlichungstag und Bekanntmachung des Hinweises auf die Patenterteilung:
**26.09.2001 Patentblatt 2001/39**

(51) Int Cl.[7]: **C07K 16/00**, A61K 39/395, G01N 33/577

(21) Anmeldenummer: **95926394.8**

(22) Anmeldetag: **06.07.1995**

(86) Internationale Anmeldenummer:
**PCT/EP95/02626**

(87) Internationale Veröffentlichungsnummer:
**WO 96/02574 (01.02.1996 Gazette 1996/06)**

(54) **VERFAHREN ZUR MODIFIZIERUNG DER STABILITÄT VON ANTIKÖRPERN**

PROCESS FOR MODIFYING THE STABILITY OF ANTIBODIES

PROCEDE DE MODIFICATION DE LA STABILITE DES ANTICORPS

(84) Benannte Vertragsstaaten:
**AT BE CH DE DK ES FR GB GR IE IT LI LU NL PT SE**

(30) Priorität: **15.07.1994 DE 4425115**

(43) Veröffentlichungstag der Anmeldung:
**07.05.1997 Patentblatt 1997/19**

(73) Patentinhaber: **Roche Diagnostics GmbH 68305 Mannheim (DE)**

(72) Erfinder:
• **STEIPE, Boris**
  **D-82131 Gauting (DE)**
• **STEINBACHER, Stefan**
  **D-82661 Lenggries (DE)**

(74) Vertreter: **Schreiner, Siegfried, Dr.
Roche Diagnostics GmbH,
Werk Penzberg,
Abt. GE-TB,
Postfach 11 52
82372 Penzberg (DE)**

(56) Entgegenhaltungen:
**WO-A-94/29350**

• **THE JOURNAL OF IMMUNOLOGY, Bd. 151, Nr. 12, 15.Dezember 1993 BALTIMORE, MD, VSA, Seiten 6954-6961, M. RODRIGUES ET AL. 'Engineering Fab' fragments for efficient F(ab)2 formation in Escherichia coli and for improved in vivo stability.'**

• **PROTEIN ENGINEERING, Bd. 7, Nr. 5, Mai 1994 OXFORD, GROSSBRITANNIEN, Seiten 697-704, Y. REITER ET AL. 'Engineering interchain disulfide bonds into conserved framework regions of Fv fragments: Improved biochemical characteristics of recombinant immunotoxins containing disulfide-stabilized Fv.'**

• **MOLECULAR IMMUNOLOGY, Bd. 28, Nr. 4-5, April 1991 OXFORD, GROSSBRITANNIEN, Seiten 489-498, E. PADLAN 'A possible procedure for reducing the immunogenicity of antibody variable domains while preserving theirligand-binding properties.'**

• **PROTEIN ENGINEERING, Bd. 7, Nr. 6, Juni 1994 OXFORD, GROSSBRITANNIEN, Seiten 805-814, G. STUDNICKA ET AL. 'Human-engineered monoclonal antibodies retain full specific binding activity by preserving non-CDR complementarity-modulating residues.'**

• **MOLECULAR IMMUNOLOGY, Bd. 30, Nr. 1, Januar 1993 OXFORD, GROSSBRITANNIEN, Seiten 105-108, S. ANGAL ET AL. 'A single amino acid substitution abolishes the heterogeneity of chimeric mouse/human (IgG4) antibody.'**

• **TRENDS IN BIOTECHNOLOGY, Bd. 7, Nr. 11, November 1989 CAMBRIDGE, GROSSBRITANNIEN, Seiten 304-310, B. GREEN ET AL. 'Catalytic monoclonal antibodies: tailor-made, enzyme-like catalysts for chemical reactions.'**

• **PROTEIN ENGINEERING, Bd. 8, Nr. 1, Januar 1995 OXFORD, GROSSBRITANNIEN, Seiten 81-89, A. KNAPPIK ET AL. 'Engineered turns of a recombinant antibody improve its in vivo folding.'**

SEQUENZPROTOKOLL

(1) ALLGEMEINE ANGABEN:

(i) ANMELDER:

(A) NAME: BOEHRINGER MANNHEIM GMBH

(B) STRASSE: Sandhofer Str. 116
(C) ORT: Mannheim
(E) LAND: Deutschland
(F) POSTLEITZAHL: D-68305
(G) TELEFON: 08856/60-3446
(H) TELEFAX: 08856/60-3451

(ii) BEZEICHNUNG DER ERFINDUNG: Verfahren zur Modifizierung der Stabilitaet
     von Antikoerpern

(iii) ANZAHL DER SEQUENZEN: 10

(iv) COMPUTER-LESBARE FASSUNG:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30B (EPA)

(vi) DATEN DER URANMELDUNG:

(A) ANMELDENUMMER: DE P 44 25 115.7
(B) ANMELDETAG: 15-JUL-1994

(2) ANGABEN ZU SEQ ID NO: 1:

(i) SEQUENZKENNZEICHEN:

(A) LÄNGE: 113 Aminosäuren
(B) ART: Aminosäure
(C) STRANGFORM:
(D) TOPOLOGIE: linear

(ii) ART DES MOLEKÜLS: Protein

(xi) SEQUENZBESCHREIBUNG: SEQ ID NO: 1:

```
Glu Val Gln Leu Val Glu Ser Gly Gly Gly Leu Val Lys Pro Gly Gly
1               5               10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
            35                  40                  45

Gly Trp Ile Tyr Asn Gly Gly Asp Thr Tyr Tyr Ala Asp Ser Val Lys
        50                  55                  60
```

```
Gly Arg Phe Thr Ile Ser Arg Asp Thr Ser Lys Asn Thr Leu Tyr Leu
65                  70                  75                  80
```

```
Gln Met Arg Ala Glu Asp Thr Ala Val Tyr Tyr Cys Ala Arg Gly Gly
                85                  90                  95
```

```
Gly Gly Gly Tyr Asp Tyr Trp Gly Gln Gly Thr Leu Val Thr Val Ser
            100                 105                 110
```

```
Ser
```

(2) ANGABEN ZU SEQ ID NO: 2:

    (i) SEQUENZKENNZEICHEN:

        (A) LÄNGE: 113 Aminosäuren
        (B) ART: Aminosäure
        (C) STRANGFORM:
        (D) TOPOLOGIE: linear

    (ii) ART DES MOLEKÜLS: Protein

    (ix) MERKMAL:

        (A) NAME/SCHLÜSSEL: Region
        (B) LAGE:99..100
        (D) SONSTIGE ANGABEN:/product= "OTHER"
           /note= "Xxa in den Positionen 99 und 100 bedeutet-keine Aminosaeure-"

    (xi) SEQUENZBESCHREIBUNG: SEQ ID NO: 2:

```
Glu Val Gln Leu Gln Gln Ser Gly Gly Glu Leu Val Lys Pro Gly Ala
1                   5                   10                  15
```

```
Ser Val Lys Leu Ser Cys Lys Ala Ser Gly Tyr Thr Phe Thr Ser Tyr
                20                  25                  30
```

```
Tyr Met His Trp Val Lys Gln Arg Pro Gly Lys Gly Leu Glu Trp Ile
            35                  40                  45
```

```
Gly Arg Ile Asn Gly Ser Gly Gly Thr Asn Tyr Asn Glu Lys Phe Lys
        50                  55                  60
```

```
Gly Lys Ala Thr Leu Thr Arg Asp Lys Ser Ser Ser Thr Ala Tyr Leu
65                  70              75                      80
```

```
Gln Leu Thr Ser Glu Asp Ser Ala Val Tyr Tyr Cys Ala Arg Gly Gly
                85              90                  95
```

```
Tyr Tyr Xaa Xaa Asp Tyr Trp Gly Gln Gly Thr Thr Val Thr Val Ser
                100              105              110
```

```
Ser
```

(2) ANGABEN ZU SEQ ID NO: 3:

   (i) SEQUENZKENNZEICHEN:

      (A) LÄNGE: 109 Aminosäuren
      (B) ART: Aminosäure
      (C) STRANGFORM:
      (D) TOPOLOGIE: linear

   (ii) ART DES MOLEKÜLS: Protein

   (xi) SEQUENZBESCHREIBUNG: SEQ ID NO: 3:

```
Asp Ile Gln Met Thr Gln Ser Pro Ser Ser Leu Ser Ala Ser Val Gly
1                   5               10                  15
```

```
Asp Arg Val Thr Ile Thr Cys Arg Ala Ser Gln Ser Ile Ser Ser Tyr
                20              25                  30
```

```
Leu Ala Trp Tyr Gln Gln Lys Pro Gly Lys Ala Pro Lys Leu Leu Ile
            35              40              45
```

```
Tyr Asp Ala Ser Asn Leu Glu Ser Gly Val Pro Ser Arg Phe Ser Gly
        50              55                  60
```

```
Ser Gly Ser Gly Thr Asp Phe Thr Leu Thr Ile Ser Ser Leu Gln Pro
65                  70              75                      80
```

```
Glu Asp Phe Ala Thr Tyr Tyr Cys Gln Gln Tyr Tyr Ser Leu Pro Tyr
                85              90                  95
```

```
Thr Phe Gly Gln Gly Thr Lys Val Glu Ile Lys Arg Thr
            100                 105
```

(2) ANGABEN ZU SEQ ID NO: 4:

   (i) SEQUENZKENNZEICHEN:

      (A) LÄNGE: 108 Aminosäuren
      (B) ART: Aminosäure
      (C) STRANGFORM:
      (D) TOPOLOGIE: linear

   (ii) ART DES MOLEKÜLS: Protein

   (xi) SEQUENZBESCHREIBUNG: SEQ ID NO: 4:

```
Asp Ile Val Met Thr Gln Ser Pro Ala Ser Leu Ser Ala Ser Leu Gly
1               5               10                  15


Glu Arg Val Thr Ile Thr Cys Arg Ala Ser Gln Ser Val Ser Ser Tyr
            20                  25                  30


Leu His Trp Tyr Gln Gln Lys Pro Gly Gln Ser Pro Lys Leu Leu Ile
            35                  40                  45


Tyr Arg Ala Ser Asn Leu Ala Ser Gly Val Pro Asp Arg Phe Ser Gly
    50                  55                  60


Ser Gly Ser Gly Thr Asp Phe Thr Leu Thr Ile Ser Ser Val Glu Ala
65                  70                  75                  80


Glu Asp Leu Ala Thr Tyr Tyr Cys Gln Gln Ser Asn Ser Tyr Pro Tyr
                85                  90                  95


Thr Phe Gly Gly Gly Thr Lys Leu Glu Ile Lys Arg
            100                 105
```

(2) ANGABEN ZU SEQ ID NO: 5:

   (i) SEQUENZKENNZEICHEN:

      (A) LÄNGE: 109 Aminosäuren
      (B) ART: Aminosäure
      (C) STRANGFORM:
      (D) TOPOLOGIE: linear

(ii) ART DES MOLEKÜLS: Protein

(ix) MERKMAL:

    (A) NAME/SCHLÜSSEL: Region
    (B) LAGE:10
    (D) SONSTIGE ANGABEN:/product= "OTHER"
        /note= "Xxa in der Position 10 bedeutet - keine Aminosaeure-"

(Xi) SEQUENZBESCHREIBUNG: SEQ ID NO: 5:

```
Gln Ser Glu Leu Thr Gln Pro Pro Ser Xaa Val Ser Val Ser Pro Gly
1               5               10              15


Gln Thr Val Thr Ile Ser Cys Ser Gly Asp Ser Leu Gly Ile Gly Tyr
            20              25              30


Val Ser Trp Tyr Gln Gln Lys Pro Gly Gln Ala Pro Lys Leu Val Ile
        35              40              45


Tyr Asp Asp Asn Lys Arg Pro Ser Gly Ile Pro Asp Arg Phe Ser Gly
    50              55              60


Ser Lys Ser Gly Asn Thr Ala Ser Leu Thr Ile Ser Gly Leu Gln Ala
65              70              75              80


Glu Asp Glu Ala Asp Tyr Tyr Cys Gln Ser Trp Asp Ser Ser Ser Val
            85              90              95


Val Phe Gly Gly Gly Thr Lys Leu Thr Val Gly Gln Pro
            100             105
```

(2) ANGABEN ZU SEQ ID NO: 6:

    (i) SEQUENZKENNZEICHEN:

        (A) LÄNGE: 109 Aminosäuren
        (B) ART: Aminosäure
        (C) STRANGFORM:
        (D) TOPOLOGIE: linear

    (ii) ART DES MOLEKÜLS: Protein

    (ix) MERKMAL:

        (A) NAME/SCHLÜSSEL: Region

(B) LAGE:10
(D) SONSTIGE ANGABEN:/product= "OTHER"
/note= "Xxa in der Position 10 bedeutet -keine Aminosaeure-"

(xi) SEQUENZBESCHREIBUNG: SEQ ID NO: 6:

```
Gln Ala Val Val Thr Gln Glu Ser Ala Xaa Leu Thr Thr Ser Pro Gly
1               5                   10                  15

Glu Thr Val Thr Leu Thr Cys Arg Ser Ser Thr Gly Ala Val Thr Tyr
            20                  25                  30

Ala Asn Trp Val Gln Glu Lys Pro Asp His Leu Phe Thr Gly Leu Ile
            35                  40                  45

Gly Gly Thr Asn Asn Arg Ala Pro Gly Val Pro Ala Arg Phe Ser Gly
        50                  55                  60

Ser Leu Ile Gly Asp Lys Ala Ala Leu Thr Ile Thr Gly Ala Gln Thr
65                  70                  75                  80

Glu Asp Glu Ala Ile Tyr Phe Cys Ala Leu Trp Tyr Ser Asn His Trp
                85                  90                  95

        Val Phe Gly Gly Gly Thr Lys Leu Thr Val Gly Gln Pro
                        100                 105
```

(2) ANGABEN ZU SEQ ID NO: 7:

(i) SEQUENZKENNZEICHEN:

(A) LÄNGE: 34 Basenpaare
(B) ART: Nucleotid
(C) STRANGFORM: Einzelstrang
(D) TOPOLOGIE: linear

(ii) ART DES MOLEKÜLS: cDNA

(xi) SEQUENZBESCHREIBUNG: SEQ ID NO: 7:

GGTAACACGT TCACCCAGTG ATACAGACAG AGAG                                34

(2) ANGABEN ZU SEQ ID NO: 8:

(i) SEQUENZKENNZEICHEN:

    (A) LÄNGE: 33 Basenpaare
    (B) ART: Nucleotid
    (C) STRANGFORM: Einzelstrang
    (D) TOPOLOGIE: linear

(ii) ART DES MOLEKÜLS: cDNA

(xi) SEQUENZBESCHREIBUNG: SEQ ID NO: 8:

```
CTGATACCAC GCCAGGTAGT TTTTCTGGTT ACC                    33
```

(2) ANGABEN ZU SEQ ID NO: 9:

(i) SEQUENZKENNZEICHEN:

    (A) LÄNGE: 34 Basenpaare
    (B) ART: Nucleotid
    (C) STRANGFORM: Einzelstrang
    (D) TOPOLOGIE: linear

(ii) ART DES MOLEKÜLS: cDNA

(xi) SEQUENZBESCHREIBUNG: SEQ ID NO: 9:

```
ACCGCTACCG CTACCCGAGA AACGGTCCGG AACA                   34
```

(2) ANGABEN ZU SEQ ID NO: 10:

(i) SEQUENZKENNZEICHEN:

    (A) LÄNGE: 33 Basenpaare
    (B) ART: Nucleotid
    (C) STRANGFORM: Einzelstrang
    (D) TOPOLOGIE: linear

(ii) ART DES MOLEKÜLS: cDNA

(xi) SEQUENZBESCHREIBUNG: SEQ ID NO: 10:

```
CGGGTAAGAG TGGTCCTGTT GACAGTAGTA AAC                    33
```

**Beschreibung**

Die Erfindung betrifft ein Verfahren zur Modifizierung der Stabilität von Antikörpern (AK) und deren Verwendung, insbesondere in der Diagnostik und Therapeutik.

Die AK-Biotechnologie ist ein stark expandierendes Feld mit Schwerpunkten in der Diagnostik (*in vitro:* z.B. Antigennachweis, *in vivo:* z.B. Imaging), in der Therapie (dort besonders humanisierte AK mit verlängerter Serum-Halbwertszeit und verringerter Immunogenizität) und in der Toxikologie (z.B. Anti-Digoxin Antikörper als spezifisches Antidot bei Herzglykosid-Überdosierung). Weitere Einsatzgebiete sind in Entwicklung bei der Induktion von Transplantattoleranz (z.B. durch anti-CD4 AK), bei der Immuntherapie (z.B. CAMPATH bei Non-Hodgkin Lymphom) und bei katalytischen AK, die besonders stereo- und regioselektive Katalyse ermöglichen.

Natürliche Antikörpersequenzen sind nicht auf Stabilität optimiert, gentechnisch erzeugte Sequenzhybride (z.B. humanisierte AK, oder single-chain Fv Fragmente) sind häufig erheblich destabilisiert. Die Folgen davon können beispielsweise sein:

- erschwerte Rückfaltung
- Denaturierung: (I) Abbau und (II) Immunogenizität schon bei 37° *in vivo*
- verschlechterte Avidität
- Aggregation und Wirkungsverlust bei Lagerung

Zur Stabilisierung von Antikörpern in Lösungen ist es beispielsweise bekannt, Proteine aus der DNAJ-Proteinfamilie (EP-A 0 556 726) oder aus der HSP90-Proteinfamilie zuzusetzen (EP-A 0 551 916). Dagegen ist bisher kein Verfahren bekannt, mit dem durch gezielte Mutationen der Aminosäuresequenz Antikörper stabilisiert werden können. Es ist zwar theoretisch möglich, in Antikörpern eine Vielzahl von Punktmutationen anzubringen und diese Mutanten auf Stabilität zu screenen. Es hat sich jedoch bei anderen Proteinen gezeigt, daß lediglich eine von $10^3$ - $10^4$ Mutanten eine verbesserte Stabilität zeigt. Solche Screeningverfahren sind damit sehr aufwendig und im übrigen beschränkt auf Proteine, die identifizierbare Funktionen, wie beispielsweise enzymatische Aktivität, besitzen (Rollence, 1988; Chen, 1989; Turner 1992; Risse, 1992; Arase, 1993).

Die Gene der variablen Domänen von Immunglobulinen haben sich durch multiple Genduplikationen und Mutationen im Laufe ihrer Entwicklung vielfältig verändert. Sie sind auf die Fähigkeit der selektiven und hochaffinen Bindung von Antikörpern optimiert (Tonegawa, 1983; Berek, 1988; French, 1989). Bei diesem Prozeß wurden die Sequenzen, welche für die Domänen codieren, zufällig mutiert und diejenigen B-Zellen selektiert und propagiert, welche verbesserte Antigenbindung zeigen (Berek, 1993). Obwohl die Optimierung der Antigenbindefähigkeit eine dominierende Rolle spielt, ist die Qualität eines Antikörpers von der Summe einer Vielzahl von Faktoren, wie Antigenaffinität, Domänenstabilität, Interaktion zwischen der schweren und leichten Kette, der variablen und konstanten Domäne, Proteaseempfindlichkeit und der Möglichkeit des Exports und der Sekretion der Antikörper aus der Zelle abhängig. Natürliche Antikörper sind demnach nicht zwangsläufig stabilitätsoptimiert.

Aus Frisch (1994) ist bekannt, daß ein humanes $V_k$-Protein nach einem Austausch von Cystein 23, wodurch die Ausbildung der Cystein 23/Cystein 88-Disulfidbrücke verhindert wird, destabilisiert ist. Diese Destabilisierung kann teilweise wieder rückgängig gemacht werden durch einen Austausch von Tryptophan 32 gegen Histidin. Dabei handelt es sich allerdings um ein Zufallsergebnis, welches überdies nicht mit der Lehre der Erfindung vereinbar ist.

Der Grund hierfür liegt darin, daß das von Frisch beschriebene $V_k$-Protein REI kein $V_k$-Domänen-Fragment eines natürlich vorkommenden Antikörpers ist, sondern ein Protein, welches als solches in einer Myelom-Zellinie überexprimiert wird. REI ist ein Protein, das sich in seiner Zusammensetzung stark von $V_k$-Domänen, die Fragmente von natürlich vorkommenden Antikörpern sind, unterscheidet. REI hat beispielsweise unübliche Aminosäuren in Position 50 (E) und 92 (Q). Auf Grund der räumlichen Anordnung der Aminosäuren kann sich vermutlich eine Salzbrücke zwischen E 50 und H 32 sowie eine Wasserstoffbrückenbildung zwischen Q 92 und H 32 ausbilden. Eine solche Wasserstoffbrückenbindung, die in natürlichen Antikörpern nicht vorkommt, stabilisiert dann dieses $V_k$-Protein.

Aufgabe der Erfindung ist es, ein Verfahren zur Verfügung zu stellen, mit dem die Stabilität von Antikörpern so modifiziert werden kann, daß diese Antikörper gezielt stabilisiert, destabilisiert oder nach destabilisierenden Maßnahmen, wie beispielsweise Entfernung der Disulfidbrücken, restabilisiert werden können.

Gegenstand der Erfindung ist ein Verfahren zur Herstellung eines stabilitätsverbesserten fünktionellen Antikörpers, fünktionellen Derivats oder Fragments davon in einem eukaryontischen oder prokaryontischen Mikroorganismus durch Transformation mit einem Expressionsvektor, der ein rekombinantes Gen enthält, welches für das genannte Immunglobulin, Derivat oder Fragment codiert, dadurch gekennzeichnet, daß

a) das Gen mindestens einer der variablen Domänen des Immunglobulins mit den Konsensustabellen 1 - 6 verglichen und dabei die Tabelle ausgewählt wird, welche die höchste Homologie zu dieser Domäne zeigt,

b) im Gen dieser variablen Domäne mindestens ein Codon einer Aminosäure ersetzt wird, und zwar

aa) falls diese Aminosäure nicht in ihrer Position in dieser ausgewählten Tabelle genannt ist, durch ein Codon für eine der genannten Aminosäuren und/oder

bb) falls diese Aminosäure in ihrer Position in der ausgewählten Tabelle genannt ist, durch ein Codon für eine der genannten Aminosäuren mit einer größeren Häufigkeit

c) den prokaryontischen oder eukaryontischen Mikroorganismus mit dem so modifizierten Gen transformiert und den Antikörper, das Fragment oder Derivat mit der gewünschten Aktivität exprimiert.

Falls erforderlich, kann der Antikörper aus dem Mikroorganismus nach den dem Fachmann geläufigen Methoden isoliert und ggf. gereinigt werden.

In einer bevorzugten Ausführungsform der Erfindung wird das Verfahren in der Weise durchgeführt, daß

a) im Gen der variablen Domäne der schweren Kette des Menschen mindestens ein Codon für eine Aminosäure ersetzt wird, und zwar

aa) falls diese Aminosäure nicht in ihrer Position in Tabelle 1 genannt ist, durch ein Codon für eine der genannten Aminosäuren und/oder

ab) falls diese Aminosäure in ihrer Position in Tabelle 1 genannt ist, durch ein Codon für eine der genannten Aminosäure mit einer größeren Häufigkeit,

b) im Gen der variablen Domäne der schweren Kette der Maus

ba) falls diese Aminosäure nicht in ihrer Position in Tabelle 2genannt ist, durch ein Codon für eine der genannten Aminosäuren und/oder

bb) falls die Aminosäure in ihrer Position in Tabelle 2 genannt ist, durch ein Codon für eine der genannten Aminosäuren mit einer größeren Häufigkeit,

c) im Gen der variablen Domäne der leichten Kette des Kappa-Typs des Menschen

ca) falls diese Aminosäure nicht in ihrer Position in Tabelle 3genannt ist, durch ein Codon für eine der genannten Aminosäuren und/oder

cb) falls die Aminosäure in ihrer Position in Tabelle 3 genannt ist, durch ein Codon für eine der genannten Aminosäuren mit einer größeren Häufigkeit,

d) im Gen der variablen Domäne der leichten Kette des Kappa-Typs der Maus

da) falls diese Aminosäure nicht in ihrer Position in Tabelle 4 genannt ist, durch ein Codon für eine der genannten Aminosäuren und/oder

db) falls die Aminosäure in ihrer Position in Tabelle 4 genannt ist, durch ein Codon für eine der genannten Aminosäuren mit einer größeren Häufigkeit,

e) im Gen der variablen Domäne der leichten Kette des λ-Typs des Menschen

ea) falls diese Aminosäure nicht in ihrer Position in Tabelle 5 genannt ist, durch ein Codon für eine der genannten Aminosäuren und/oder

eb) falls die Aminosäure in ihrer Position in Tabelle 5 genannt ist, durch ein Codon für eine der genannten Aminosäuren mit einer größeren Häufigkeit,

f) im Gen der variablen Domäne der leichten Kette des λ-Typs der Maus

fa) falls diese Aminosäure nicht in ihrer Position in Tabelle 6 genannt ist, durch ein Codon für eine der genannten Aminosäuren und/oder

fb) falls die Aminosäure in ihrer Position in Tabelle 6 genannt ist, durch ein Codon für eine der genannten Aminosäuren mit einer größeren Häufigkeit,

g) und der prokaryontische oder eukaryontische Organismus transformiert und der Antikörper, das Fragment oder Derivat mit der gewünschten Aktivität exprimiert wird.

Die Anwendung des erfindungsgemäßen Verfahrens erfolgt in der Weise, daß der Antikörper, der stabilisiert werden soll, zunächst sequenziert wird und die Sequenz seiner Domänen mit den in den Tabellen 1 - 6 genannten Konsensussequenzen oder den Sequenzen von Kabat (1991) verglichen wird. Die Aminosäurepositionen werden bei maximaler Homologie der Sequenzen definiert. Anschließend können -zweckmäßig durch Mutagenese- ein oder mehrere Codons gemäß der Erfindung modifiziert werden. Es zeigt sich, daß bereits der gezielte Austausch eines Codons eine deutliche Veränderung der Stabilität eines Antikörpers bewirken kann. Vorzugsweise werden jedoch zwei, drei oder mehr Codons modifiziert. Eine Obergrenze für die Zahl der Austausche wird dann erreicht, wenn andere Eigenschaften des Antikörpers, die für den gewünschten Einsatzzweck wichtig sind (z. B. Affinität, Proteasestabilität, Selektivität) negativ beeinflußt werden.

Das Vorgehen soll an einem Beispiel verdeutlicht werden:

Die Aminosäurepositionen werden zunächst durch Sequenzvergleich (maximale Homologie) mit den Tabellen 1 - 6 oder mit den Tabellen von Kabat (1991) ermittelt.

Für einen humanen Antikörper, dessen Stabilität nicht optimal ist, wird gefunden, daß an Position 15 der schweren Kette die Aminosäure H steht. Aus Tabelle 1 ist zu entnehmen, daß für Position 15 G oder S bevorzugt sind. Es ist demnach vorteilhaft H durch S oder besonders bevorzugt durch G zu ersetzen. Falls gefunden wird, daß in Position 16 in diesem Antikörper sich die Aminosäure A befindet, so ist es bevorzugt, A durch Q, R oder G zu ersetzen. Offensichtlich ist es besonders vorteilhaft, A durch G zu ersetzen.

Falls der Antikörper z. B. nach Position 35 einen Einschub von einer oder zwei Aminosäuren besitzt, so ist es bevorzugt, mindestens eine dieser Aminosäuren zu deletieren (35 a/35 b durch "-" zu ersetzen). Analoges gilt für die anderen optionalen Einschübe. Die Tabellen sind also so zu verstehen, daß die Aminosäuren an den Positionen, die mit a, b etc. bezeichnet sind (z. B. 35a, 35b), zur Stabilisierung des Antikörpers vorzugsweise deletiert werden (also durch die Aminosäure "-" ersetzt werden). Für die Position 100 b in Tabelle 1 bedeutet dies, daß zur Stabilisierung beispielsweise eine nicht genannte Aminosäure durch G oder S ersetzt werden kann. Vorzugsweise wird diese Aminosäure jedoch deletiert. Ebenso vorteilhaft ist es aber auch, G oder S an dieser Position zu deletieren.

Um einen Antikörper nach dem erfindungsgemäßen Verfahren zu stabilisieren und dennoch seine anderen Eigenschaften, wie insbesondere die Affinität zum Antigen, zu erhalten, werden vorzugsweise Aminosäuren ausgetauscht, die diese Eigenschaften möglichst nicht beeinträchtigen. Aus diesem Grund ist es bevorzugt, keine Austausche in den antigenbindenden loops oder CDRs durchzuführen.

Die Herstellung der Antikörperderivate und Fragmente kann nach den dem Fachmann geläufigen Methoden für die Herstellung von rekombinanten Proteinen erfolgen. Derartige Verfahren sind beispielsweise in EP-B 0 125 023 und EP-B 0 120 694 und bei S.L. Morrison et al. (1984) beschrieben.

Zur Herstellung der gemäß der Erfindung modifizierten Antikörper, kann beispielsweise die komplette DNA der variablen Domäne synthetisiert werden (über Oligonukleotidsynthese, wie beispielsweise in Sinha et al., NAR 12 (1984), 4539 - 4557 beschrieben). Die Oligonukleotide können über PCR gekoppelt werden, wie beispielsweise bei Innis, Ed. PCR protocols, Academic Press (1990) und Better et al., J. Biol. Chem. 267 (1992), 16712 - 16118 beschrieben. Die Klonierung und Expression erfolgt über Standardverfahren, wie beispielsweise in Ausubel et al., Eds. Current protocols in Molecular Biology, John Wiley and Sons, New York (1989) und in Robinson et al., Hum. Antibod. Hybridomas 2 (1991) 84 - 93 beschrieben. Die Überprüfung der spezifischen Antigenbindeaktivität kann beispielsweise über einen Kompetitionstest erfolgen, wie in Harlow et al., Eds. Antibodies: A Laboratory Manual, Chapter 14, Cold Spring Harbor Laboratory, Cold Spring Harbor (1988) and Munson et al., Anal. Biochem. 407 (1980), 220 - 239 beschrieben.

Geeignete Wirtsorganismen sind beispielsweise CHO-Zellen, Lymphozytenzellinien, die keine Immunglobuline produzieren, Hefe, Insektenzellen und Prokaryonten, wie E.coli.

Ein weiterer Gegenstand der Erfindung ist ein derartiges Verfahren, bei dem das Protein in einem prokaryontischen Organismus (z. B. E.coli) als denaturierte Inclusion bodies isoliert wird und nach dem Fachmann geläufigen Verfahren (vgl. z. B. EP-A 0 364 926) aktiviert wird. Dabei kann überraschenderweise die Aktivierung auch unter reduzierenden Bedingungen erfolgen.

Ein weiterer Gegenstand der Erfindung ist ein Verfahren, bei dem der Antikörper gemäß der Erfindung so stabilisiert wird, daß er mit der gewünschten Aktivität im Cytosol biologisch aktiv entsteht und daraus direkt und in aktiver Form isoliert werden kann.

Das erfindungsgemäße Verfahren verbessert die Stabilität von Antikörpern und Antikörperfragmenten für alle oben angesprochenen Anwendungsgebiete. Darüberhinaus sind gemäß der Erfindung neue stabile Antikörpervarianten herstellbar, die bisher nicht stabil erhältlich waren, wie z. B. disulfidbrückenfreie AK oder katalytische Antikörper, die zum Einsatz unter unphysiologischen Bedingungen geeignet sind. Katalytische Antikörper und deren Verwendung sind beispielsweise in CIBA Foundation Symposium on Catalytic antibodies, London, 1990, Ed. Chadwick D.J., Marsh J., Band 159, Wiley and Sons, Chichester beschrieben.

Stabilisierte disulfidbrückenfreie Antikörper werden dadurch erhalten, daß die Cysteine, welche Disulfidbrücken bilden, durch andere Aminosäuren ersetzt werden und mindestens eine, vorzugsweise zwei oder mehr Aminosäuren durch stabilitätsvermittelnde Aminosäuren ersetzt werden.

Vorzugsweise sind solche Antikörper chimäre, humanisierte, nicht humane oder humane, einer β-Lymphozyten Expression zuordenbare Antikörper (kein REI Protein).

Ein weiterer Gegenstand der Erfindung ist ein Verfahren zur Herstellung von nicht-disruptiven destabilisierten Antikörper, die beispielsweise vorteilhaft verwendet werden können, wenn eine rasche Pharmokinetik benötigt wird. Um solche Antikörper zu erhalten, muß konsequenterweiser mindestens ein Aminosäureaustausch in entgegengesetzter Weise, wie oben beschrieben, durchgeführt werden. Dies bedeutet, daß eine Aminosäure mit größerer Häufigkeit durch eine Aminosäure mit geringerer Häufigkeit ersetzt wird.

Als Antikörperfragmente geeignet sind beispielsweise Fab, Fab', F(ab')$_2$, single chain-Antikörper, Fv oder einzelne variable Domänen. Diese Fragmente können ebenso an weitere Substanzen, beispielsweise zu Immuntoxinen, gekoppelt sein.

Besonders vorteilhaft ist das erfindungsgemäße Verfahren zur Verbesserung der Stabilität von single chain F$_V$-Regionen von Antikörpern, insbesondere zur Stabilitätsverbesserung von single chain-Immunotoxinen. In solchen single chain-antigenbindenden Proteinen sind die leichte und die schwere Kette auf verschiedene Weise aneinander gebunden. Diese Bindung ist beispielsweise über eine Disulfidbrücke, über kovalente Bindungen oder über eine Zinkkomplexbindung möglich. Derartige single chain-Proteine und deren Kopplung sind beispielsweise in Brinkmann et al., P.N.A.S. 89 (1992), 3075 - 3079 (Kopplung über einen Peptidlinker), bei Brinkmann et al., P.N.A.S. 90 (1993), 7536 - 7542 (zusätzliche Disulfidbrücke) beschrieben. Weitere Immunotoxine und Bindemöglichkeiten sind in der WO 91/09871, WO 91/12820, WO 91/16069 beschrieben.

Ein weiterer Vorteil der Erfindung besteht darin, daß scF$_v$ (single chain F$_v$, Hybridproteine aus V$_H$ und V$_L$ Domäne, die durch ein unstrukturiertes Oligopeptid verbunden sind) gemäß der Erfindung stabil und in einer wenig immunogenen Form herstellbar sind. Die üblicherweise verwendeten Linkerpeptide (S.H. Jung (1994), R. Glockshuber (1990) der scFvs führen häufig zu Aggregationsproblemen und sind potentielle Immunogene. Die kovalente Verknüpfung von V$_H$ und V$_L$ Domänen kann dagegen auch durch eine intermolekulare Cystinbrücke erfolgen, solche zusätzlichen Cysteine führten aber bislang zu einer erheblichen Beeinträchtigung der Faltungsausbeute durch die Möglichkeit der Ausbildung falscher Disulfidbrücken. Durch das erfindungsgemäße Verfahren können die konservierten Cysteine an den Positionen 23/88 (leichte Kette), 22/99 (schwere Kette) durch Mutagenese ersetzt werden und die Stabilität der Antikörper gemäß den erfindungsgemäßen Verfahren wiederhergestellt oder verbessert werden. Damit ist die falsche Ausbildung von Disulfidbrücken ausgeschlossen. Das erfindungsgemäße Verfahren ist deshalb für die therapeutische Nutzung rekombinanter Antikörperhybride von großer Bedeutung.

Außerdem bieten Antikörper die Möglichkeit, durch Selektion im Immunsystem auf eine große Zahl von Effektorfunktionen maßgeschneidert zu werden. Dieses natürliche *protein engineering* System ist in seiner Effizienz unerreicht. Durch die zytoplasmatische Expression spezieller funktioneller Antikörperdomänen können solche Effektorfunktionen in die Zellen eingeführt werden. Vorteilhaft sind Anwendungen, die auf die Modulation der Aktivität zellulärer Proteine hinauslaufen. Dies kann beispielsweise durch Stabilisierung des Zielproteins durch Protein-Antikörper Komplexbildung geschehen. Damit kann eine Veränderung der Degradationskinetik erreicht werden. Auch allosterische Effektorwirkungen sind möglich. Die Approximation zweier Effektoren durch Ausbildung und Stabilisierung eines ternären Komplexes schafft eine weitere Möglichkeit der Beeinflußung von Stoffwechselwegen beispielsweise durch artifizielle Multi-Enzymkomplexe oder die lokale Erhöhung von Metabolitkonzentrationen an induzierbaren Operatoren. Besonders vorteilhaft ist aber die zytoplasmatische Expression katalytischer Antikörper und die damit verbundene Möglichkeit auf katalytische Effizienz zu selektieren. Eine cytoplasmarische Expression von funktionellen Antikörpern ist für gemäß der Erfindung stabilisierte AK auf einfache Weise möglich.

Ein weiterer Vorteil des erfindungsgemäßen Verfahrens besteht darin, daß Antikörper, die nach der Expression inaktiv entstehen unter reduzierenden Bedingungen (z. B. DTE, DTT, Glutathion) aktiviert werden können.

Die gemäß der Erfindung stabilisierten Antikörper können vorteilhaft in allen Einsatzgebieten von Antikörpern verwendet werden, beispielsweise in der Therapeutik von Krebs und Infektionen, als Immuntoxin, zum Drug-targeting und in der Gentherapie. Ebenso ist eine Verwendung zum Imaging und in der Diagnostik, beispielsweise zur Analyse von antigenbindenden Substanzen vorteilhaft.

Besonders vorteilhaft ist das erfindungsgemäße Verfahren zur Stabilisierung von Antikörpern, die aus anderen Gründen bereits modifiziert sind, wie beispielsweise humanisierte oder chimäre Antikörper. Durch diese Modifikation der Aminosäuren kann sich eine Destabilisierung ergeben, die durch das erfindungsgemäße Verfahren bei einer zusätzlichen Modifikation dieser Antikörper außerhalb der CDR-Bereiche dazu führt, daß die ursprüngliche Stabilität der Antikörper wieder hergestellt oder sogar verbessert wird.

**Methode zur Analyse der Sequenzdatenbank und zum Auffinden der Tabellen 1 - 6 (*canonical sequence approximation*)**

Die Erfindung geht davon aus, daß die natürlich vorkommenden Immunglobulinsequenzen eine kanonische Sammlung von Sequenzen ist, die in ihrer Summe kompatibel für alle Aspekte der Antikörperfunktionen sein sollte.

Es sollte die in der Natur am häufigsten an einer Position beobachtete Aminosäure auch diejenige sein, die ein Protein am besten stabilisiert; dies gilt besonders bei Proteinen, bei denen natürlicherweise auf Stabilität und nicht auf spezielle Funktionen selektiert wird.

An den Positionen

35, 37, 39, 44, 45, 47, 50, 91, 95, 100j, 100k, 101 und 103

der schweren Kette

und

1, 32, 34, 36, 38, 43, 44, 46, 49, 50, 87, 89, 95, 96 und 98

der leichten Kette des Menschen sind die Aminosäuren an wichtigen Wechselwirkungen bei der Ausbildung von heterodimeren Fv Fragmenten beteiligt; dort wird nicht primär auf Stabilität selektiert. Ist das Ziel die Verbesserung der Dimerisierungseigenschaften, sind auch an diesen Positionen die häufigsten Aminosäuren zu wählen, ist das Ziel die Verbesserung der Stabilität können auch die zweit- oder dritthäufigsten Aminosäuren gewählt werden.

Die natürlichen Häufigkeiten der Aminosäuren sind aus einer Stichprobe der Immunglobulin Datenbank (Kabat, 1991) bestimmt. Es ist wichtig, daß diese Stichprobe die tatsächlichen Verhältnisse gut repräsentiert. Aus diesem Grund ist auch offensichtlich, daß die Tabellen 1-6 nach Verfügbarkeit weiterer, zusätzlicher Daten unter Umständen geringfügig modifiziert werden können. Der Theorie entsprechend können Vorhersagen für die Verteilung innerhalb einer Spezies (z.B. Mensch oder Maus) und innerhalb eines Subtyps (z.B. Kappa oder Lambda) angegeben werden.

Manche eng verwandte Sequenzen sind aus methodischen Gründen in der Datenbank überrepräsentiert. Dies führt dazu, daß die Datenbank ohne weitere Modifikation keine geeignete Zufallsstichprobe darstellt. Aus diesem Grund werden aus der Datenbank lediglich die weitgehend vollständigen Sequenzen ausgewählt um Probleme mit der Definition eines Sequenzabstands zwischen Sequenzen die nur bruchstückhaft bekannt sind, zu vermeiden. Ausgewählt werden Sequenzen, von denen mehr als 75% der Positionen bekannt sind, entsprechend nicht mehr als 30 fehlenden Positionen für die leichten Ketten und nicht mehr als 33 fehlenden Positionen bei den schweren Ketten. Damit gehen als Sequenzen der Kabat Datenbank in die weitere Analyse ein:

| Protein | Anzahl |
|---|---|
| $V_L$-Kappa, Maus | 731 von 1068 Sequenzen |
| $V_L$-Kappa, Mensch | 127 von 319 Sequenzen |
| $V_L$-Lambda, Mensch | 82 von 148 Sequenzen |
| $V_L$-Lambda, Maus | 63 von 74 Sequenzen |
| $V_H$, Mensch | 178 von 325 Sequenzen |
| $V_H$, Maus | 883 von 1620 Sequenzen |

In diesen Sequenzen werden alle paarweisen Sequenzabstände berechnet und analysiert. Typischerweise ergibt sich eine zweigipflige Verteilung, mit einem Maximum um den mittleren Abstand aller Sequenzen dieses Subtyps. Diese Verteilung der Sequenzabstände kann verwendet werden um den Effekt von Stichprobenfehlern zu verringern: wenn die Sequenzen der natürlichen Verteilung einen bestimmten mittleren Abstand und eine bestimmte Verteilung der Abstände besitzen, werden Stichprobenfehler verringert, wenn eine Stichprobe aus der Datenbank unter den selben Randbedingungen der Abstandsverteilung gezogen wird. Die verwendeten Randbedingungen sind:

| Protein | Mindestabstand | Höchstabstand |
|---|---|---|
| VL-Kappa, Maus | 25 | 57 |
| $V_L$-Kappa, Mensch | 25 | 57 |
| $V_L$-Lambda, Mensch | 33 | 65 |

(fortgesetzt)

| Protein | Mindestabstand | Höchstabstand |
|---|---|---|
| $V_L$-Lambda, Maus | 8 | 26 |
| $V_H$, Mensch | 37 | 77 |
| $V_H$, Maus | 37 | 77 |

Es werden dazu zufällig Sequenzen aus der Datenbank ausgewählt und überprüft, ob sie einem Mindestabstand und einem Höchstabstand zu den vorher ausgewählten Sequenzen genügen. Ist dies der Fall, werden sie der neuen Stichprobe zugeordnet. Dies wird wiederholt, bis alle Sequenzen auf ihre Eignung Mitglied der Stichprobe zu sein, überprüft worden sind. Typischerweise werden dabei zwischen 5 und 20% der Sequenzen der Datenbank ausgewählt. Wird diese Auswahl oft (hier 500 mal) wiederholt, wird jede einzelne Sequenz in der Stichprobe repräsentiert sein, allerdings mit unterschiedlicher Häufigkeit, entsprechend ihres Abstandes zu den anderen Sequenzen. Schließlich werden die Aminosäurehäufigkeiten für die einzelnen Positionen aus dieser neuen Stichprobe bestimmt.

Für die hier ermittelten Häufigkeiten wurde nach dem oben beschriebenen *resampling*- Verfahren vorgegangen, wobei Aminosäuren dieren Häufigkeit unter 0.1 (=10%) beträgt, in den Tabellen nicht aufgelistet wurden.

Die nachfolgenden Beispiele, Abbildungen, Tabellen und das Sequenzprotokoll beschreiben die Erfindung näher:

## Beschreibung der Abbildungen

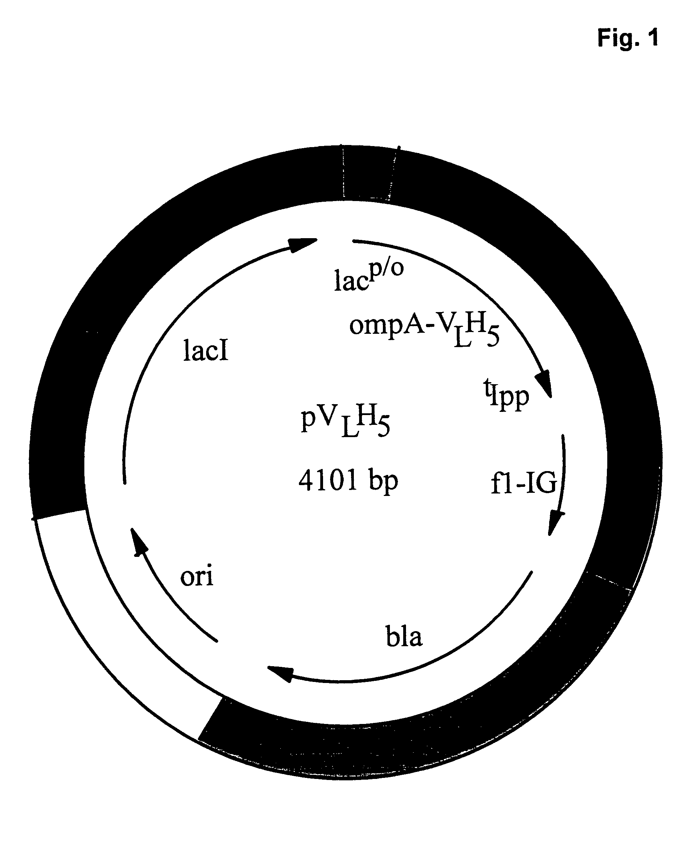

<u>Fig. 1</u>: Expressionplasmid $pV_LH_5$ ($lac^{p/o}$: Promotor/Operator Region des lac-Gens; ompA $V_LH_5$ codierender Bereich für die VL-Domäne mit Signalsequenz des *outer membrane protein A*; $tl_{pp}$ Terminator; f1-IG: F1-Phagenreplikationsursprung; bla: Gen der b-Laktamase; ori: Plasmid-Replikationursprung; lacI: Gen des lac-Repressors). Die Darstellung ist nicht maßstabsgetreu.

(Das Plasmid entspricht im wesentlichen dem in der EP-B 0 324 162 beschriebenen Plasmid. Dort ist auch die Expression von Antikörpern mit diesem Plasmid beschrieben.)

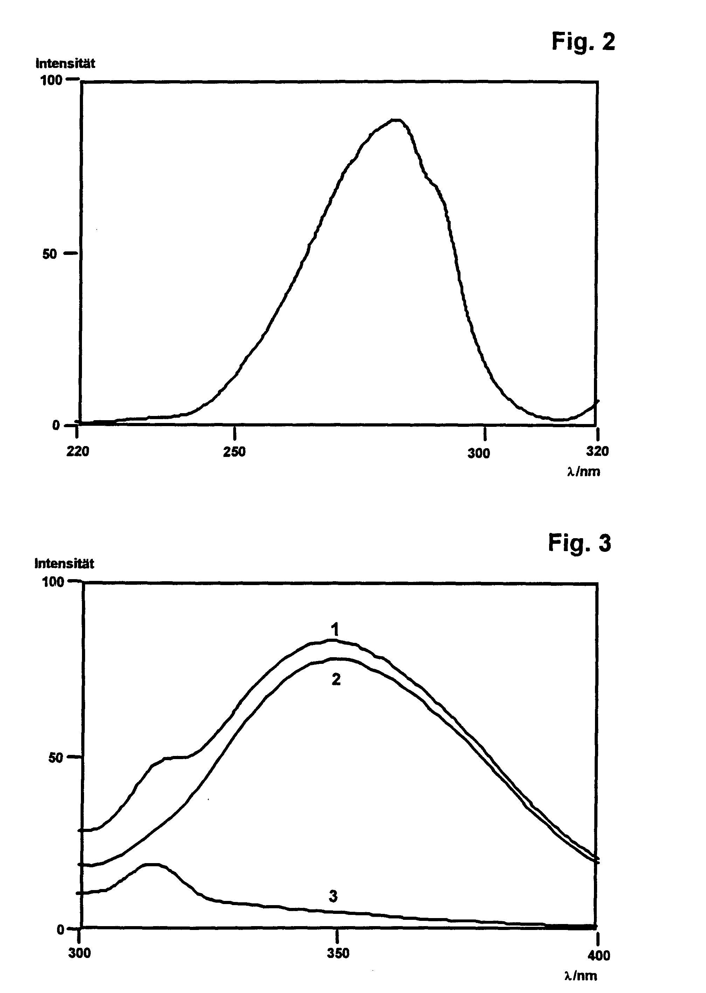

<u>Fig. 2</u>: Excitationsspektrum.des VL-Proteins. Die Emission wird bei $I_{Em}$ = 360 nm gemessen Proteinkonzentraion: 2 mM in PBS. Die Intensität ist in willkürlichen Einheiten angegeben.

<u>Fig. 3</u>: Fluroreszenzspektrum des gefalteten (1), des entfalteten (3) VL-Proteins und das Differenzspektrum (2). Anregungswellenlänge $I_{Ex}$ = 284 nm. Proteinkonzentraion: 2 mM in PBS. Die Intensität ist in willkürlichen Einheiten angegeben.

## Beispiele

## Bakterien und Phagen

| E. coli K12-Stämme | |
|---|---|
| CJ 236 | dutl, ungl, thi-1, relAI [pCJ 105 (Cam[1]), F] (Geisselsoder *et al*., 1987) von Bio-Rad Laboratories GmbH, München |
| JM 83 | ara, D(lac-pro AB), strA, thi-1 [F801acZM15] (Yanisch-Perron *et al*. 1985) |
| JM 109 | recAl, supE44, endAl, hsd R17, gyrA96, relA1,thi_(lac-proAB) (Yanisch-Perron *et al.* 1985) |
| **Bakteriophage** | |
| M13K07 | Helferphage (Vieira & Messing, 1987) von Deutsche Pharmacia GmbH, Freiburg |

## Plasmide

Das Plasmid $pV_LH_5$ codiert für die VL-Domäne des Antikörpers McPC603 unter der Kontrolle des lac-Promotors/Operators. Zur Reinigung des Proteins zur Homo-genität in einem Schritt durch Chromatographie an immobilisierten Zinkionen sind die beiden C-terminalen Aminosäuren Arginin (108) und Alanin (109) durch fünf Histidinreste ersetzt. Im Gegensatz zur Wildtyp-Sequenz befindet sich an Position 106 statt eines Isoleucin- ein Leucin-Rest.

Zur Sekretion ins Periplasma ist vor den codierenden Bereich von $V_L$ die Signalsequenz des *outer membrane protein A* geschaltet (Skerra & Plückthun 1989).

**Oligodesoxynukleotide**

Die verwendeten Oligodesoxynukleotide wurden nach dem Phosphoramiditverfahren an einem DNA-Synthesegerät 380A der Applied Biosystems GmbH, Weiterstadt hergestellt.

(SEQ ID NO:7)

LD 38  (A15L)    5'- GGT AAC ACG TTC ACC CAG TGA TAC AGA  CAG AGA G

(SEQ ID NO:8)

LD 40  (F32T)    5'- CTG ATA CCA CGC CAG GTA GTT TTT CTG GTT ACC

(SEQ ID NO:9)

LD 42  (T63S)    5'- ACC GCT ACC GCT ACC CGA GAA ACG GTC  CGG AAC A

(SEQ ID NO:10)

LD 44  (N90Q)    5'- CGG GTA AGA GTG GTC CTG TTG ACA GTA  GTA AAC

**Vermehrung von E. coli Kulturen**

**(Maniatis *et al*. 1982)**

Nach Inkubation bei 37°C unter Schütteln bei 180 bis 200 Upm für 14 bis 20 Stunden wird eine dichte Übernachtkultur erhalten. Die Zelldichte wird durch Messung der OD600 ermittlet. Zur Selektion auf plasmidtragende Stämme wird das Medium mit einem geeigneten Antibiotikum versetzt.

LB-Medium:   10 g/l Bacto-Trypton
             5 g/l Bacto-Yeast-Extract
             5 g/l NaCL
             2.5 ml/l 1 M NaOH

**Transformation von E. coli mit Plasmid-DNA (Hanahan, 1983)**

Kompetente Zellen

Zur Transformation werden E. coli Zellen kompetent gemacht.
In einem 250 ml Erlenmeyerkolben werden 20 ml TYM-Medium mit 0.5 ml einer stationären Übernachtkultur des verwendeten E. coli-Stammes angeimpft und bei 37°C bis zu einer $OD_{600}$ von 0.2 bis 0.8 inkubiert. Diese Kultur wird zu 100 ml TYM-Medium gegeben. Nach Wachstum zu einer $OD_{600}$ von 0.5 bis 0.9 wird mit TYM-Medium auf 500 ml Gesamtvolumen aufgefüllt und weiter inkubiert. Beim Erreichen einer $OD_{600}$ von 0.6 wird die Bakterien-suspension abgekühlt.
Die Bakterien werden bei 4200 Upm und 4°C 15 Minuten abzentrifugiert, die Überstände abgegossen und die Pellets in insgesamt 80 ml TfB I-Puffer resuspendiert, abzentrifugiert, die Überstände abgegossen und die Pellets in insgesamt 16 ml eiskaltem TfB II-Puffer resuspendiert.

TYM:      20 g/l Bacto-Trypton
          5 g/l Bacto-Yeast-Extract

        100 mM NaCl
        10 mM MgSO$_4$

TfB I:        30 mM KOAc
              50 mM MnCl$_2$
              100 mM KCl
              10 mM CaCl$_2$
              15 % (v/v) Glycerin

TfB II:       75 mM CaCl$_2$
              10 mM KCl
              10 mM NaMOPS pH 7.0
              15 % (v/v) Glycerin

Transformation

Die Plasmid-DNA wird in einem Volumen von 30 µl Wasser zugegeben und mit der Bakteriensuspension gut vermischt. Nach 60 Minuten auf Eis erfolgt ein Hitze-schock von 115 Sekunden bei 37°C. Nach einer Minute auf Eis werden 800 µl LB-Medium zugegeben, die Bakteriensuspension in ein 10 ml Kulturröhrchen überführt und 60 Minuten bei etwa 180 Upm und 37°C inkubiert. Der gesamte Transformationsansatz wird auf eine LB-Platte mit Antibiotikum gegossen und über 12 bis 16 Stunden bei 37°C inkubiert.

**Mutagenese**

Die Mutagenese erfolgt unter Verwendung der Puffer des Muta-Gene™ *in vitro* Mutagenesis Kits (Bio Rad Laboratories GmbH, DE) nach Kunkel 1985, Geisseloder et al., 1987, Vieira und Messing , 1987.

Erzeugung von Doppelmutationen durch Umklonieren von DNA-Fragmenten

Im Zuge der Bestimmung der Konformationsstabilität der Einzelmutanten (2.3) erwiesen sich u.a. Ala15Leu, Asn90Gln und Phe32Tyr als stabilisierend. Um die Additivität der stabilisierenden Effekte zu untersuchen, wurden die Doppelmutanten Ala15Leu/Asn90Gln und Ala15Leu/Phe32Tyr hergestellt.
Die Doppelmutanten wurden durch Umklonieren von DNA-Fragmenten bereits erzeugter Einzelmutanten dargestellt. Nach einem Restriktionsverdau wurden die Fragmente durch Agarosegelelektrophorese getrennt, die gewünschten Frag-mente aus der Agarose ausgeschnitten, die DNA daraus isoliert und in geeigneter Weise ligiert.

Ala15Leu/Phe32Tyr:

Der Verdau der Plasmid-DNA der Mutanten Ala15Leu und Phe32Tyr mit der Restriktionsendonuklease Bst EII lieferte je zwei Fragmente. Ein 3232 bp-Fragment enthält die Basen der Aminosäure 32, ein Fragment von 870 bp die für Aminosäure 15. Als Differenz der freien Enthalpie der Entfaltung zum unmodifizierten Antikörper wurden 22,6 kJ/mol (theor. 20.8) gefunden.

Ala15Leu/Asn90Gln:

Der Verdau der Plasmid-DNA der Mutanten Als15Leu und Asn90Gln mit der Restriktionsendonuklease Xmn I liefert je zwei Fragmente. Ein 2991 bp-Fragment enthält die Basen der Aminosäure 15, ein Fragment von 1110 bp die für Aminosäure 90. Als Differenz der freien Enthalpie der Entfaltung zum unmodifizierten Antikörper wurden 23,9 kJ/mol (theor. 23.6) gefunden.

**Expression rekombinanter V$_L$-Domänen und Aufarbeitung**

Die Expression (Skerra und Plückthun 1988) der VL-Proteine erfolgt unter der Kontrolle des lac-Operators/ Repressors in *Escherichia coli*, wobei die Induktion der Expression mit IPTG erfolgt. Vor den kodierenden Bereich des Proteins ist die Signalsequenz des *outer mebrane protein A* (ompA) geschaltet, welche die Sekretion des Proteins ins Periplasma bewirkt und dabei durch die *E. coli* eigene Signalpeptidase abgespalten wird. Die Sekretions ins Periplasma ermöglicht aufgrund des dort herrschenden höheren (oxidierenden) Redoxpotentials die Ausbildung der zentralen Disulfid-brücke und damit die korrekte Faltung des VL-Proteins, welche im Cytoplasma aufgrund des niedrigeren (reduzierenden) Redoxpotentials nicht möglich ist (Gilbert 1990).

Durch einen selektiven Aufschluß des Periplasmas (1 M NaCl/ 1mM EDTA/ 50 mM Tris/HCl pH8.0) läßt sich das Protein im Gemisch mit anderen periplasmatischen Proteinen bequem isolieren. Die fünf C-terminalen Histidinreste, welche anstelle der Aminosäuren 108 und 109 vorhanden sind, ermöglichen eine einfache Reinigung des Proteins zur Homogenität in einem Schritt durch Chromatographie an immobilisierten Zinkionen (Hochuli *et al*. 1988)).

10 Liter LB-Medium werden mit 200 ml stationärer Übernachtkultur E. coli JM 83/ $pV_LH_5$ angeimpft und mit 10 ml AMP-Stammlösung versetz. Die Kultur wird belüftet und bei Raumtemperatur bis $OD_{600}$ 0.7 inkubiert (etwa vier Stunden).

Zur Induktion der $V_LH_5$ -Expression unter der Kontrolle des lac-Operators/ Repressors werden 5 ml 1 M IPTG-Lösung zugesetzt, sowie 5 ml AMP-Stamm-lösung die den Verlust an Selektionsantibiotikum ausgleichen, welcher dadurch entsteht, daß lysierte Bakterien b-Lactamase aus dem Periplasma ins Medium freisetzen.

Es wird weitere 3 Stunden inkubiert. Zur Ernte der Bakterien wird die Kultur zu je etwa 430 ml in 500 ml Zentrifugenbecher für den Rotor JA-10 einer Beckman-Zenrtifuge abgefüllt und bei 6000 Upm je 10 Minuten zentrifugiert. Mit 6 Zentrifu-genbechern sind 4 Zentrifugengänge erforderlich.

Nach Abgießen der Überstände werden typischerweise um die 30 Gramm Bakterienpellets erhalten.

Periplasmaaufschluß

Pro Gramm Zellen werden 2 ml Periplasmaaufschlußpuffer zugegeben, die Bakterien bei 4°C unter Rühren resuspendiert und mindestens eine Stunde kräftig weitergerührt. Anschließend wird die milchig-hellbraune Suspension in Zentrifu-genbecher für den Rotor JA-20 einer Beckman-Zentrifuge überführt und die Sphäroblasten durch 20 Minuten Zentrifugieren bei 20 000 Upm, 4 °C abgetrennt. Der klare, leicht gelbe Überstand mit dem rekombinanten VL-Protein wird in 50 ml Falcon-Gefäße überführt und bis zur weiteren Verwendung bei 4°C gelagert.

Chromatographie an immobilisierten Zinkionen (Hochuli *et al. 1988.* Lindner et al. 1992)

Die fünf C-terminalen Histidinreste der VL-Domäne erhöhen die Bindung des Proteins an immobilisiert Zinkionen so stark, daß es in einem Schritt zur Homogenität gereinigt werden kann. Das Zink ist dabei an einen Iminodiacetat-Chelatliganden komplexiert, der seinerseits an Sepharose gekoppelt ist. Die Histidin-Reste des Proteins fungieren nun als Komplexliganden am Zink und werden so an das Säulenmaterial gebunden. Die Elution kann mit Imidazol erfolgen, welches die Histidin-Reste am Zink verdrängt.

Vorbereitung der Säule

Zur Regeneration der Säule (etwa 5 ml Chelating-Sepharose Fast Flow der Deutschen Pharmacia GmbH, Freiburg) wird zunächst mit 50 ml Regenera-tionspuffer und dann mit 20 ml Wasser gespült, um komplexiertes Zink und damit eventuell noch gebundene Proteine zu entfernen. Anschließend wird die Säule mit 15 ml Zinkchloridlösung (1 mg/ml),15 ml Wasser und 50 ml Säulenäquilibrierungspuffer gespült.

Chromatographie

Die Chromatographie wird mit einer Flußrate von etwa 0.7 bis 1 ml/min durchge-führt, wobei Fraktionen von je 10 Minuten gesammelt werden.

Nach dem Auftragen des Periplasmaaufschlusses (typischerweise etwa 70 ml) wird mit Säulenäquilibrie-rungspuffer gespült, bis die $OD_{280}$ auf den Nullwert zurückgegangen ist. Durch Spülen mit 10 mM Imidazol in Säulenäquilibrierungs-puffer werden schwach gebundene Proteine eluiert. Die Elution der $V_LH_5$- Domäne erfolgt in einem linearen Gradienten von 10 bis 300 mM Imidazol in Säulen-äquilibrierungspuffer und einem Gesamtvolumen von 200 ml bei etwa 70 mM Imidazol. Die Reinheit des Proteins wird durch SDS-Polyacrylamid-Gelelektrophorese überprüft.

| Periplasmaaufschlußpuffer: | 1 M NaCl |
| | 1 mM EDTA |
| | 50 mM Tris/HCl pH 8.0 |
| Säulenäquilibrierungspuffer: | 1 M NaCl |
| | 50 mM Tris/HCl pH 8.0 |
| Regenerationspuffer: | 1 M NaCl |
| | 50 mM EDTA |

50 mM Tris/HCl pH 8.0

Anschließend wir die Proteinlösung, welche die gewünschte Menge an VL-Protein enthält (etwa 1 bis 2 mg) zweimal gegen das hundertfache Volumen des entsprechenden Puffers dialysiert.

### Ermittlung von Denaturierungskurven

Zur Ermittlung von Denaturierungskurven wird das VL-Protein gegen PBS dialysiert und auf eine Konzentration von 0.2 mM (2.5 mg/ml; M = 12.4 kDa) gebracht. Um Präzipitate und andere Partikel aus der Proteinlösung zu entfernen, wird diese vor Verwendung 10 Minuten in einer Kühlzentrifunge Sigma 2K15 zentrifugiert und der Überstand in ein neues 1.5 ml Eppendorfreaktionsgefäß überführt. Je 5 µl dieser Proteinlösung werden mit einer 10 µl Hamilton-Spritze in einem 5 ml Reagenzglas vorgelegt, mit 500 µl Denaturierungspuffer versetzt, das Reagenzglas mit einem Silikonstopfen verschlossen und über Nacht bei 20°C inkubiert.

Als Denaturierungspuffer dienen Guanidiniumchlorid-Lösungen in PBS im Konzentrationsbereich von 0 bis 5 M. Bis 2 M erfolgt der Konzentrationsanstieg in Schritten von 0.1 M, darüber in Schritten von 0.2 M.

| Geräteparameter: | |
|---|---|
| Anregungswellenlänge | $1_{Ex}$ = 284 nm |
| Emisionwellenlänge | $1_{Em}$ = 360 nm |
| Excitationsschlitzbreite | 2 nm |
| Emissionsschlitzbreite | 10 nm |

### 2.3 Die Analyse von Denaturierungskurven zur Bestimmung der freien Enthalpie der Entfaltung

In der Gegenwart denaturierender Verbindungen verlieren Proteine ihre native Konformation und damit ihre biologische Funktion. Harnstoff und Guanidinium-chlorid sind hierbei besonders wirksam. Viele lösliche globuläre Proteine können durch diese reversibel entfaltet werden und zeigen ein einfaches Zweizustands-verhalten. Dies läßt sich durch Vergleich kalorimetrischer Daten ($DH_{cal}$.) mit den entsprechenden van t'Hoff-Enthalpien ($DH_{van}$ t'Hoff), welche aus der Temperatur-abhängigkeit der Gleichgewichtskonstanten ermittelt werden können, aufzeigen. Das Verhältnis beider sollte eins betragen. Es wurde gezeigt, daß Abweichungen hiervon für eine große Zahl von Eindomänenproteinen sehr gering sind. Dies zeigt, daß eventuell auftretende Intermediate thermodynamisch instabil sind. Man kann sie deshalb vernachlässigen und die Denaturierung demzufolge als einen kooperativen Übergang zwischen zwei makroskopischen Zuständen ansehen, dem gefalteten (F) und dem entfalteten (U) (Privalov 1979).

Das entfaltete Protein stellt dabei ein Ensemble rasch ineinander überführbarer Konformerer dar, welche die gleiche oder eine sehr ähnliche Energie besitzen. Im Idealfall würde der denaturierte Zustand ein Zufallsknäuel bilden, d.h. die Rotation um Bindungen sollte völlig frei und unabhängig von der Rotation aller Nachbarn erfolgen. Da die Wechselwirkungen zwischen dem Lösungsmittel, den Hauptkettenatomen des Proteins und den 20 verschiedenen Seitenketten der Aminosäuren nicht in gleicher Weise ideal sein können, wird ein vom idealen Zufallsknäuel abweichendes Verhalten erwartet (Tanford 1970). Der Raum-anspruch einer realen Kette wird außerdem zur Aufrechterhaltung kurzreichender Wechselwirkungen beitragen (Flory 1969).

Es wird aber davon ausgegangen, daß in konzentrierter Guanidiniumchloridlösung eine "komplette" Entfaltung erreicht wird, die mit der durch andere Denaturierungsmittel erreichten übereinstimmt (Privalov 1979, Creighton 1978). Mit den Methoden der NMR-Spektroskopie (Wüthrich 1986) kann man jedoch auch Struktur und Dynamik individueller Gruppen im denaturierten Zustand untersuchen. Dieser erscheint als eine große Zahl signifikant unterschiedlicher, "polymorpher" Konformere in raschem Gleichgewicht (Dobson et al. 1985). Die Untersuchung von Proteinmutanten gibt einen starken Hinweis darauf, daß sich die Kompaktheit des denaturierten Zustands, ebenso wie dessen Energie, durch einzelne Mutationen stark beeinflussen läßt (Shortle 1992).

Unter Anwendung des Zweizustandsmodells läßt sich die thermodynamische Gleichgewichtskontante K definieren:

$$F \rightleftharpoons U \qquad (1)$$

$$K_u = \frac{[U]}{[F]} \qquad (2)$$

Die freie Enthalpie der Entfaltung ergibt sich daraus zu:

$$\Delta G_u = - RT \ln K_u \tag{3}$$

Das Verhältnis [U]/[F] läßt sich mit zahlreichen spektroskopischen Meßmethoden bestimmen, welche einen Unterschied in den Eigenschaften des nativen und entfalteten Zustands erfassen, z.B. Cirkulardichroismus, UV-Absorption oder Fluo-reszenzspektroskopie. Letztere Methode gilt als empfindlichste, man benötigt die geringsten Proteinmengen. Ein Meßsignal I setzt sich dabei additiv aus den An-teilen des gefalteten ($I_f$) und entfalteten ($I_u$) zusammen:

$$I = I_u + I_f$$

Diese sind den im Gleichgewicht jeweils vorhandenen Konzentrationen [F] und [U] proportional. Mit cf und $c_u$ als Proportionalitätskonstanten, welche durch die stoffspezifischen, spektroskopischen Eigenschaften der beiden Zustände gegeben sind, erhält man:

$$I = c_f[F] + c_u[U] \tag{4}$$

Mit der Stoffmengenbilanz ([P]: Proteinkonzentration)

$$[P] = [F] + [U] \tag{5}$$

erhält man durch Division von (4) und (5) nach Umformung unter Berücksichtigung von $c_f[P] = I_f o$ und $c_u [P] = I_u o$, welche die Signalintesitäten der vollständig ge- bzw. entfalteten Zustand repräsentieren:

$$\frac{[U]}{[F]} = \frac{I - I_f^o}{I^o - I} \tag{6}$$

Es kann vorkommen, daß die Signalintensitäten des vollständig ge- bzw. entfalteten Zustand von der Konzentration des Denaturierungsmittel abhängen. Dies läßt sich durch einen linearen Zusammenhang in guter Näherung berücksichtigen. Im Fall der VL-Domänen der vorliegenden Arbeit trifft dies für den entfalteten Zustand zu, dies wird mit (7) berücksichtigt.

$$I_u^o ([GdmHCl]) = I_u^o - a \cdot [GdmHCl] \tag{7}$$

Bei der Entfaltung eines Proteins mit einem Denaturierungsmittel wie Guani-diniumchlorid wird die Stabiliät des Proteins mit zunehmender Denaturierungs-mittelkonzentration verringert, mit anderen Worten $\Delta G_u$ wird kleiner. Es wird bei der Analyse von Denaturierungskurven für Proteine die ein Zweizustandsverhalten zeigen davon ausgegangen, daß zwischen der Konzentration des Denaturierungs-mittels und $\Delta G_u$ ein linearer Zusammenhang besteht (Pace 1986, Alonso und Dill 1991).

$$\Delta G_u = \Delta G_u^o - m \cdot [D] \tag{8}$$

Unter Anwendung von (3) und (6) läßt sich $\Delta G_u$ im Konzentrationsbereich des Denaturierungsmittels berechnen, in dem sowohl die gefaltete als auch die entfaltete Form in erfassbaren Konzentrationen vorliegen. Die freie Enthalpie der Entfaltung in Abwesenheit des Denaturierungsmittels erhält man dann durch lineare Extrapolation auf null molar Denaturierungsmittels, wobei die Gültigkeit von (8) zugrundegelegt ist.

Eine zweite Möglichkeit der Auswertung besteht darin, unter Verwendung von (2), (3), (6), (7) und (8) einen Ausdruck für die Signalintensität in Abhängigkeit der Parameter abzuleiten (9) und diese durch Anpassung des theoretischen Kurvenverlaufs an die Meßwerte nach dem Prizip der kleinsten Fehlerquadrate zu erhalten.

$$I=I_u^o-a\cdot[\text{GdmHCl}]+\frac{I_f^o-I_u^o+a\cdot[\text{GdmHCl}]}{1+e^{\frac{m\cdot[\text{GdmHCl}]-\Delta G_u^o}{RT}}} \qquad (9)$$

Als Parameter treten folgende Größen auf: $I_u^o$, $I_f^o$, $\Delta G_f^u$, a und m.

Zur Erstellung der Denaturierungskurven der VL-Mutanten wird die Fluoreszenz als Meßgröße herangezogen. Die Fluoreszenz des VL-Proteins geht hauptsächlich auf den einzigen Tryptophanrest 35 zurück, der im Proteininneren gegen die zentrale Disulfidbrücke gepackt ist. Im Zuge der Entfaltung kommt der Tryptophan-rest in eine hydrophilere Umgebung und die Wechselwirkung mit der Disulfid-brücke geht verloren. Die sehr geringe Fluoreszenz des gefalteten Proteins ist auf die fluoreszenzlöschende Wirkung der Disulfidbrücke zurückzuführen (Cowgill 1967).

Fig. 2 zeigt die Fluoreszenzspektren des gefalteten und des entfalteten Zustands (2 mM Protein in PBS mit 0 M bzw. 5 M GdmHCl 20°C), sowie das Differenzspektrum beider. Im Zuge der Entfaltung nimmt die Proteinfluoreszenz mit einem Maximum bei 350 nm etwa um den Faktor 16 zu. Die Fluoreszenz erweist sich also im vorliegenden Fall als eine ideale Meßgröße, da sie mit der Entfaltung des Proteins eine deutliche Änderung erfährt. Fig. 3 zeigt ein Anregungsspektrum, die Fluoreszenz bei 350 wird in Abhängigkeit der Anregungswellenlänge er-mittelt. Man erkennt ein ausgeprägtes Maximum bei 280 nm.

PBS   4 mM $KH_2PO_4$
      16 mM $Na_2HPO_4$
      115 mM NaCl
      der pH-Wert beträgt 7.4

Es wurden meist mehrere Messungen durchgeführt, die Daten wurden dabei durch Mittelung der nach (10) (aus(5),(6) und (7)) normierten Werte erhalten.

$$\frac{[U]}{[P]}=\frac{I-I_f^o}{I_u^o-a\cdot[\text{GdmHCl}]-I_f^o} \qquad (10)$$

Aus den erhaltenen Parametern kann man auch diejenige Konzentration errechnen, bei der die Hälfte des Proteins entfaltet vorliegt, den Denaturierungs-mittelpunkt $[\text{GdmHCl}]_{1/2}$. Es gilt hier $DG_u = 0$. Aus (8) erhält man (11).

$$[\text{GdmHCl}]_{1/2}=\frac{\Delta G_u^o}{m} \qquad (11)$$

Die Parameter der Einzelmessungen sind in Tabelle 7 zusammengefaßt.

Tabelle 7

| Vergleich zwischen Vorhersage und Experiment für stabilisierende Punktmutationen | | | | | |
|---|---|---|---|---|---|
| Domäne | $f_{WT}$ | $f_{mut}$ | $\Delta G^P$fold (kJ mol$^{-1}$) | Experiment | Vorhersage |
| WT | | | -13.5 | | |
| Ala15Leu | 0.082 | 0.411 | -19.2 | ++ | ++ |
| Asn90Gln | 0.047 | 0.892 | -17.9 | ++ | ++ |
| Phe32Tyr | 0.034 | 0.799 | -15.1 | + | ++ |
| Leu106Ile | 0.298 | 0.684 | -15.0 | + | + |
| Thr63Ser | 0.148 | 0.823 | -14.7 | + | ++ |
| Met21Ile | 0.278 | 0.590 | -14.5 | + | + |
| Met21Leu | 0.278 | 0.103 | -12.2 | - | - |

Bei der Expression der Proteine wurde außerdem überraschenderweise beobachtet, daß die Ausbeute im Vergleich zum Wildtyp auf etwa neun bis 26 Milligramm bei stabileren Mutanten anstieg.

### 7. <u>Literaturverzeichnis</u>

Alonso, D. O. V., Dill, K. A. (1991). Solvent Denaturation and Stabilization of Globular Proteins. *Biochemistry* **30,** 5974-5985

Birnboim, C. Doly, J. (1979). Rapid alkaline extraction procedure for screening recombinant plasmid DNA. *Nucleic Acids. Res.* **7,** 1513-1523

Cowgill, R. W. (1967). Fluorescence Quenching by Disulfide and Sulfhydryl Groups. *Biochimica et Biophysica acta* **140,** 37-44

Creighton, T. E. (1978). Experimental Studies of Protein Folding and Unfolding. *Prog. Biophys. molec. Biol.* **33,** 231-297

Devereux et al., Nucleic Acids Res. 12 (1984), 387 - 395

Dobson, C. M. Evans, P. A., Fox, R. O. (1985). In: *Structure and Motion. Membranes Nucleic Acids and Proteins*, Adenine, Guilderland, New York, 265-276

Flory, P. (1969). *Statistical Mechanics of Chain Molecules,* Wiley, New York, 432 ff.

Frisch C. et al., Biol. Chem. Hoppe-Seyler 375 (1994) 353 - 356

Geisselsoder, J., Witney, F., Yuckenberg, P. (1987). Efficient site-dircted *in vitro* mutagenesis. *Biotechniques* **5,** 786-791

Gellert, W. (Hsg.) (1984). Kleine Enzyklopädie Mathematik, 2. Auflage, Verlag Harri Deutsch, Thun Frankfurt/ M., 668 ff.

Gilbert, H. F. (1990). Molecular and cellular aspects of thiol-disulfide exchange. *Adv. Enzymol*. **63,** 69-172

Glockshuber, R. (1989). Das F$_V$-Fragment des Phosphorylcholin bindenden Antikörpers McPC603 : Expression in *Escherichia coli* und Charakterisierung. *Dissertation,* Fakultät für Chemie und Pharmazie der Ludwig-Maximilians-Universität, München

Hanahan, D. (1983). Studies of transformation of *Escherichia coli* with plasmids. *J. Mol. Biol.* **166,** 557-579

Hochuli, E., Bannworth, W., Döbeli, H., Gentz, R., Süber, D. (1988). Genetic approach to facilitate purification of recombinant proteins with a novel metal chelat adsorbent. *Bio/Technology* **6,** 1321-1325

Kaplan, B. E. (1985). The automated synthesis of oligodeoxyribonucleotides. *Trends Biotechnol.* **3,** 253-256

Kunkel, T. A. Roberts, J. D., Zakow, R. A. (1987). Rapid and efficient site-specific mutagenesis without phenotypic selection. *Methods Enzymol*. **154,** 367-382

Maniatis, T. Fritsch, E. F., Sambrook, J. (1982). *Molecular cloning: A laboratory manual*. Cold Spring Harbor Laboratory, New York

Morrison, S.L. et al., Ann. Rev. Immunol. 2 (1984) 239 - 256 Pace, C. N. (1986). Determination and Analysis od Urea and Guanidine Hydrochloride Denaturation Curves. *Methods Enzimol*. **131,** 266

Privalov, P. L., Gill, S. J. (1989). The hydrophobic effect: a reappraisal. *Pure & Appl. Chem.* **61,** 1097-1104

Shortle, D. (1992). Mutational studies of protein structures and their stabilities. *Q. Rev. Bioph.* **25,** 205-250

Skerra, A., Plückthun, A. (1988). Assembly of functional F$_V$ fragments in *Escherichia coli. Science* **240,** 1038-1041

Tanford, C. (1970). Protein Denaturation. Part C. Theoretical Models for the Mechanism of Denaturation. *Adv. Protein Chem.* **24,** 1-95

Vieira, J. Messing, J. (1987). Production of single-stranded plasmid DNA. *Methods. Enzymol.* **153,** 3-11

Wüthrich, K. (1986). *NMR of Proteins and Nucleic Acids,* Wiley, New York, 293 ff.

Yanisch-Perron, C., Vieira, J. Messing, J. (1985). Improved M13 phage cloning vectors and host strains: Nucleotide sequences ofthe M13mp18 and pUC19 vectors. *Gene* **33,** 103-119

Frisch, C. et. al., Biol. Chem. Hoppe-Seyler 375 375 (1994) 353 - 356

Rollence, M.L., Filpula, D., Pantoliano, M.W. & Bryan P.N. Crit. Rev. Biotechnol. 8, 217 - 224 (1988)

Chen, L.H. & Baldwin, T.O. Biochemistry 28, 2684 - 2689 (1989)

Turner, S.L., Ford, G.C., Mountain, A. & Moir, A. Prot. Eng. 5, 535 - 541 (1992) Risse, B., Stempfer, G., Rudolph, R., Schumacher, G., & Jaenicke, R Protein Science 1, 1710 - 1718 (1992).

Arase, A., Yomo, T., Urabe, I., Hata, Y., Katsube, Y. & Okada, H. FEBS lett., 316, 123 - 127 (1993).

Kabat, E.A., Wu, T.T., Perry, H.M., Gottesman, K.S. & Foeller, C. Sequences of Proteins of Immunological Interest, 5th ed. US Dept. Health and Human Services, Bethesda, Md. (1991)

Tonegawa, S. Nature 302, 575 - 581 (1983)

Berek, C. & Milstein, C., Immunol. Rev. 105, 5 - 26 (1988)

French, D.L., Laskov, R. & Scharff, M.D. Science, 244, 1152 - 1157 (1989)

Berek, C. & Ziegner, M. Immunol. Today 14, 400 - 404 (1993)

Jung, S.H. et al., Proteins: Structure Function and Genetics 19 (1994) 35 - 47

Glockshuber, R. et al., Biochemistry 29 (1990) 1362 - 1367

Lindner, P. et al., Methods: A comparison to Methods of Enzymology 4 (1992) 41 - 56

Tabelle 1

Variable Domänen der schweren Kette des Menschen

Ermittelte Konsensussequenz

**Tabelle 1**

```
 1    EVQLVESGGGLVKPGGSLRLSCAASGFTFSSYAMS--WVRQA
41    PGKGLEWVGWIY---NGGDTYYADSVKGRFTISRDTSKNTLYL
81    QMNSLRAEDTAVYYCARGGGGGY----------FDYWGQGTLVTVSS
```

(SEQ ID NO: zeigt die Konsensussequenz ohne Einschübe

| Position | Typ und Häufigkeit |
|---|---|
| _ 1 | E:0.505 Q:0.460 |
| _ 2 | V:0.939 |
| _ 3 | Q:0.920 |
| _ 4 | L:0.997 |
| _ 5 | V:0.680 Q:0.177 L:0.106 |
| _ 6 | E:0.638 Q:0.333 |
| _ 7 | S:0.930 |
| _ 8 | G:1.000 |
| _ 9 | G:0.566 A:0.215 P:0.177 |
| _ 10 | G:0.609 E:0.246 |
| _ 11 | L:0.637 V:0.336 |
| _12 | V:0.701 K:0.221 |
| _ 13 | K:0.455 Q:0.455 |
| _14 | P:0.964 |
| _15 | G:0.796 S:0.177 |
| _ 16 | G:0.357 R:0.189 Q:0.124 E:0.116 A:0.116 |
| _17 | S:0.803 T:0.179 |
| _ 18 | L:0.794 V:0.188 |
| _19 | R:0.574 K:0.230 S:0.164 |
| _ 20 | L:0.730 V:0.217 |
| _21 | S:0.786 T:0.214 |
| _ 22 | C:1.000 |
| _ 23 | A:0.506 K:0.228 T:0.132 |
| _ 24 | A:0.655 V:0.174 |
| _ 25 | S:0.977 |
| _ 26 | G:0.985 |
| _ 27 | F:0.578 Y:0.161 G:0.147 |
| _ 28 | T:0.585 S:0.266 |
| _ 29 | F:0.804 |
| _ 30 | S:0.761 T:0.110 |

(fortgesetzt)

| Position | Typ und Häufigkeit |
|----------|--------------------|
| _ 31 | S:0.389 D:0.167 T:0.160 |
| _ 32 | Y:0.535 S:0.124 |
| _ 33 | A:0.292 Y:0.168 W:0.127 |
| _ 34 | M:0.598 I:0.183 |
| _ 35 | S:0.310 H:0.272 N:0.112 |
| _ 35a | -:0.897 |
| _ 35b | -:0.922 |
| _ 36 | W:1.000 |
| _ 37 | V:0.784 I:0.151 |
| _ 38 | R:1.000 |
| _ 39 | Q:0.994 |
| _ 40 | A:0.648 P:0.162 |
| _41 | P:0.923 |
| _ 42 | G:0.969 |
| _ 43 | K:0.729 Q:0.156 R:0.104 |
| _ 44 | G:0.909 |
| _ 45 | L:0.959 |
| _ 46 | E:0.972 |
| _ 47 | W:0.996 |
| _ 48 | V:0.566 M:0.196 I:0.150 |
| _ 49 | G:0.510 S:0.243 A:0.197 |
| _ 50 | W:0.199 V:0.113 |
| _ 51 | I:0.807 |
| _ 52 | Y:0.211 S:0.167 N:0.115 G:0.107 |
| _52a | -:0.198 P:0.159 Y:0.128 G:0.113 |
| _ 52b | -:0.897 |
| _ 52c | -:0.927 |
| _ 53 | N:0.170 D:0.166 S:0.134 G:0.125 |
| _ 54 | G:0.402 S:0.204 |
| _ 55 | G:0.476 S:0.269 |
| _ 56 | D:0.198 S:0.183 T:0.158 N:0.143 |
| _ 57 | T:0.465 K:0.105 |
| _ 58 | Y:0.304 N:0.186 H:0.114 |
| _ 59 | Y:0.894 |
| _ 60 | A:0.656 N:0.129 |
| _ 61 | D:0.394 P:0.205 Q:0.138 |
| _ 62 | S:0.714 K:0.122 |
| _63 | V:0.590 F:0.219 L:0.154 |
| _ 64 | K:0.554 Q:0.237 |
| _ 65 | G:0.785 S:0.148 |
| _ 66 | R:0.926 |
| _ 67 | F:0.602 V:0.348 |
| _ 68 | T:0.878 |
| _ 69 | 1:0.806 M:0,111 |
| _ 70 | S:0.789 T:0.130 |
| _ 71 | R:0.597 V:0.150 |
| _ 72 | D:0.815 N:0.152 |
| _ 73 | T:0.301 N:0.284 D:0.253 |
| _ 74 | S:0.890 A:0.103 |

(fortgesetzt)

| Position | Typ und Häufigkeit |
|----------|--------------------|
| _ 75 | K:0.643 |
| _ 76 | N:0.672 S:0.221 |
| _ 77 | T:0.659 Q:0.211 |
| _ 78 | L:0.462 A:0.252 F:0.179 |
| _ 79 | Y:0.710 S:0.192 |
| _ 80 | L:0.822 M:0.169 |
| _ 81 | Q:0.573 E:0.198 |
| _ 82 | M:0.548 L:0.344 |
| _ 82a | N:0.399 S:0.300 |
| _ 82b | S:0.797 |
| _ 82c | L:0.753 V:0.202 |
| _ 83 | R:0.542 T:0.196 K:0.131 |
| _ 84 | A:0.485 P:0.191 S:0.134 |
| _ 85 | E:0.644 A:0.155 D:0.127 |
| _ 86 | D:0.972 |
| _ 87 | T:0.940 |
| _ 88 | A:0.956 |
| _ 89 | V:0.765 |
| _90 | Y:0.992 |
| _ 91 | Y:0.947 |
| _ 92 | C:0.998 |
| _ 93 | A:0.891 |
| _ 94 | R:0.681 K:0.158 |
| _ 95 | G:0.179 D:0.152 E:0.119 V:0.100 |
| _ 96 | G:0.118 P:0.101 |
| _ 97 | G:0.168 S:0.122 |
| _ 98 | G:0.132 Y:0.103 |
| _ 99 | G:0.240 A:0.111 |
| _100 | Y:0.139 S:0.127 -:0.127 G:0.120 |
| _100a | -:0.276 S:0.160 |
| _100b | -:0.379 S:0.107 G:0.101 |
| _100c | -:0.429 Y:0.110 |
| _100d | -:0.567 |
| _100e | -:0.645 Y:0.129 |
| _100f | -:0.728 Y:0.107 |
| _100g | -:0.758 Y:0.114 |
| _100h | -:0.825 |
| _100i | -:0.868 |
| _100j | -:0.481 Y:0.147 |
| _100k | F:0.475 -:0.176 M:0.160 L:0.100 |
| _101 | D:0.755 |
| _102 | Y:0.442 V:0.239 |
| _103 | W:0.967 |
| _104 | G:0.953 |
| _105 | Q:0.823 |
| _106 | G:1.000 |
| _107 | T:0.887 |
| _108 | L:0.659 T:0.194 |
| _109 | V:0.986 |

(fortgesetzt)

| Position | Typ und Häufigkeit |
|----------|--------------------|
| _110 | T:0.916 |
| _111 | V:0.969 |
| _112 | S:0.980 |
| _113 | S:0.930 |

Tabelle 2

Variable Domänen der schweren Kette der Maus

Ermittelte Konsensussequenz

```
 1  EVQLQQSGGELVKPGASVKLSCKASGYTFTSYYMH--WVKQR
41  PGKGLEWIGRINP--GSGGTNYNEKFKGKATLTRDKSSSTAYL
81  QLSSLTSEDSAVYYCARGGYY-----------YFDYWGQGTTVTVSS
```

(SEQ ID NO:2 zeigt die Konsensussequenz ohne Einschübe)

| Position | Typ und Häufigkeit |
|----------|--------------------|
| _ 1 | E:0.504 Q:0.409 |
| _ 2 | V:0.965 |
| _ 3 | Q:0.756 K:0.186 |
| _ 4 | L:0.968 |
| _ 5 | Q:0.575 V:0.227 |
| _ 6 | Q:0.563 E:0.434 |
| _ 7 | S:0.818 P:0.122 |
| _ 8 | G:0.976 |
| _ 9 | G:0.314 P:0.311 A:0.246 T:0.107 |
| _ 10 | E:0.560 G:0.353 |
| _ 11 | L:0.951 |
| _ 12 | V:0.810 |
| _ 13 | K:0.526 Q:0.248 R:0.118 |
| _ 14 | P:0.895 |
| _ 15 | G:0.883 |
| _16 | A:0.383 G:0.314 |
| _ 17 | S:0.940 |
| _ 18 | V:0.599 L:0.290 |
| _19 | K:0.738 S:0.100 |
| _ 20 | L:0.569 I:0.245 M:0.173 |
| _21 | S:0.915 |
| _ 22 | C:1.000 |
| _ 23 | K:0.528 A:0.222 T:0.121 |
| _ 24 | A:0.779 |
| _ 25 | S:0.912 |

(fortgesetzt)

| Position | Typ und Häufigkeit |
|---|---|
| _ 26 | G.0.988 |
| _ 27 | Y:0.591 F:0.380 |
| _ 28 | T:0.671 S:0.171 |
| _ 29 | F:0.858 |
| _ 30 | T:0.578 S:0.323 |
| _ 31 | S:0.351 D:0.276 N:0.122 |
| _ 32 | Y:0.723 |
| _ 33 | Y:0.312 W:0.298 G:0.163 |
| _ 34 | M:0.664 I:0.199 |
| _ 35 | H:0.300 N:0.283 S:0.181 |
| _ 35a | -:0.971 |
| _ 35b | -:0.998 |
| _ 36 | W:0.997 |
| _ 37 | V:0.909 |
| _ 38 | K:0.550 R:0.434 |
| _ 39 | Q:0.945 |
| _ 40 | R:0.384 S:0.170 A:0.143 T:0.105 |
| _ 41 | P:0.866 |
| _ 42 | G:0.750 E:0.195 |
| _ 43 | K:0.525 Q:0.321 |
| _44 | G:0.671 R:0.108 S:0.102 |
| _ 45 | L:0.981 |
| _ 46 | E:0.930 |
| _ 47 | W:0.944 |
| _ 48 | 1:0.647 V:0.176 L:0.102 |
| _ 49 | G:0.742 A:0.250 |
| _ 50 | R:0.196 Y:0.157 E:0.103 |
| _51 | I:0.921 |
| _ 52 | N:0.295 Y:0.185 S:0.147 D:0.116 R:0.101 |
| _52a | P:0,550 S:0.148 |
| _52b | -:0.893 K:0.104 |
| _52c | -:0.891 |
| _ 53 | G:0.321 N:0.190 Y:0.162 S:0.102 |
| _ 54 | S:0.310 N:0.309 G:0.222 |
| _ 55 | G:0.568 S:0.153 Y:0.107 |
| _ 56 | G:0.162 Y:0.158 S:0.149 T:0.126 N:0.117 |
| _ 57 | T:0.763 I:0.115 |
| _ 58 | N:0.295 Y:0.183 K:0.161 |
| _ 59 | Y:0.956 |
| _ 60 | N:0.536 A:0.181 |
| _ 61 | E:0.294 Q:0.197 D:0.184 P:0.150 |
| _ 62 | K:0.508 S:0.269 |
| _ 63 | F:0.607 V:0.221 L:0.131 |
| _ 64 | K:0.809 |
| _ 65 | G:0.596 D:0.174 S:0.166 |
| _ 66 | K:0.532 R:0.466 |
| _ 67 | A:0.516 F:0.341 |
| _ 68 | T:0.773 |
| _ 69 | L:0.437 I:0.417 |

(fortgesetzt)

| Position | Typ und Häufigkeit |
|----------|--------------------|
| _ 70 | T:0.590 S:0.373 |
| _ 71 | R:0.339 V:0.306 A:0.230 |
| _ 72 | D:0.895 |
| _ 73 | K:0.366 N:0.258 T:0.238 |
| _ 74 | S:0.764 A:0.123 |
| _ 75 | S:0.539 K:0.254 |
| _ 76 | S:0.585 N:0.334 |
| _ 77 | T:0.772 |
| _ 78 | A:0.514 L:0.269 V:0.168 |
| _ 79 | Y:0.868 F:0.108 |
| _ 80 | L:0.481 M:0.475 |
| _ 81 | Q:0.742E:0.137 |
| _ 82 | L:0.589 M:0.342 |
| _ 82a | S:0.525 N:0.286 |
| _82b | S:0.710 N:0.109 |
| _82c | L:0.891 V:0.101 |
| _ 83 | T:0,587 R:0.231 K:0.104 |
| _ 84 | S:0.736 |
| _ 85 | E:0.876 |
| _ 86 | D:0.981 |
| _ 87 | S:0.513 T:0.428 |
| _ 88 | A:0.941 |
| _ 89 | V:0.542 T:0.150 M:0.126 1:0.104 |
| _ 90 | Y:0.980 |
| _ 91 | Y:0.770 F:0.227 |
| _ 92 | C:0.997 |
| _ 93 | A:0.725 T:0.127 |
| _ 94 | R:0.821 |
| _ 95 | G:0.174 D:0.158 Y:0.125 |
| _ 96 | G:0.205 Y:0.150 |
| _ 97 | Y:0.242 G:0.181 |
| _ 98 | Y:0.249 -:0.208 G:0.149 |
| _ 99 | -:0.310 G:0.181 S:0.105 |
| _100 | -:0.484 S:0.110 G:0.108 |
| _100a | -:0.612 |
| _100b | -:0.768 |
| _100c | -:0.867 |
| _100d | -:0.910 |
| _100e | -:0.974 |
| _100f | -:0.986 |
| _100g | -:0.992 |
| _100h | -:0.997 |
| _100i | -:1.000 |
| _100j | Y:0.324 -:0.276 A:0.177 W:0.101 |
| _100k | F:0.590 M:0.187 -:0.108 |
| _101 | D:0.668 A:0.233 |
| _102 | Y:0.774 V:0.152 |
| _103 | W:0.986 |
| _104 | G:0.985 |

(fortgesetzt)

| Position | Typ und Häufigkeit |
|----------|-------------------|
| _105 | Q:0.834 |
| _106 | G:0.993 |
| _107 | T:0.973 |
| _108 | T:0.475 L:0.283 S:0.226 |
| _109 | V:0.695 L:0:294 |
| _110 | T:0.960 |
| _111 | V:0.987 |
| _112 | S:0.984 |
| _113 | S:0.753 A:0.233 |

Tabelle 3

Variable Domänen der leichten Kette des Kappa-Typs des Menschen

Ermittelte Konsensussequenz

```
  1 DIQMTQSPSSLSASVGDRVTITCRASQSISS------YLAWYQQKPGKAPKLLIYD
 51 ASNLESGVPSRFSGSGSGTDFTLTISSLQPEDFATYYCQQYYSLP------YTFGQ
101 GTKVEI-KRT
```

(SEQ ID NO:3 zeigt die Konsensussequenz ohne Einschübe)

| Position | Typ und Häufigkeit |
|----------|-------------------|
| _ 1 | D:0.858 E:0.133 |
| _ 2 | I:0.933 |
| _ 3 | Q:0.746 V:0.237 |
| _ 4 | M:0.754 L:0.240 |
| _ 5 | T:0.999 |
| _ 6 | Q:1.000 |
| _ 7 | S:0.985 |
| _ 8 | P:1.000 |
| _ 9 | S:0.750 |
| _ 10 | S:0.630 T:0.339 |
| _ 11 | L:0.965 |
| _ 12 | S:0.885 |
| _ 13 | A:0.683 V:0.206 L:0.111 |
| _ 14 | S:0.927 |
| _15 | V:0.741 P:0.188 |
| _ 16 | G:1.000 |
| _ 17 | D:0.763 E:0.227 |
| _ 18 | R:0.875 |
| _ 19 | V:0.742 A:0.224 |
| _ 20 | T:0.915 |

(fortgesetzt)

| Position | Typ und Häufigkeit |
|----------|---------------------|
| _ 21 | I:0.758 L:0.203 |
| _ 22 | T:0.693 S:0.193 |
| _ 23 | C:1.000 |
| _ 24 | R:0.678 Q:0.146 |
| _ 25 | A:0.801 S:0.145 |
| _ 26 | S:0.876 |
| _ 27 | Q:0.914 |
| _ 28 | S:0.569 T:0.129 |
| _ 29 | 1:0.435 V:0.390 L:0.101 |
| _ 30 | S:0.374 L:0.165 V:0.107 N:0.100 |
| _ 31 | S:0.244 N:0.214 K:0.152 Y:0.109 |
| _ 31a | -:0.717 S:0.210 |
| _ 31b | -:0.812 |
| _ 31c | -:0.811 |
| _ 31d | -:0.811 N:0.113 |
| _ 31e | -:0.855 |
| _ 31f | -:0.936 |
| _ 32 | Y:0.544 W:0.151 |
| _ 33 | L:0.930 |
| _ 34 | A:0.477 N:0.355 |
| _ 35 | W:1.000 |
| _ 36 | Y:0.909 |
| _ 37 | Q:0.907 |
| _ 38 | Q:0.985 |
| _ 39 | K:0.909 |
| _ 40 | P:0.985 |
| _ 41 | G:0.913 |
| _ 42 | K:0.669 Q:0.289 |
| _ 43 | A:0.871 |
| _44 | P:0.998 |
| _ 45 | K:0.576 R:0.189 N:0.105 |
| _ 46 | L:0.786 |
| _47 | L:0.996 |
| _ 48 | I:0.975 |
| _ 49 | Y:0.912 |
| _ 50 | D:0.263 A:0.255 G:0.176 |
| _ 51 | A:0.772 V:0.113 |
| _ 52 | S:0.951 |
| _ 53 | N:0.389 S:0.292 T:0.252 |
| _ 54 | L:0.693 R:0.307 |
| _ 55 | E:0.335 Q:0.219 A:0.168 |
| _ 56 | S:0.490 T:0.333 |
| _ 57 | G:0.981 |
| _ 58 | V:0.788 I:0.183 |
| _ 59 | P:0.992 |
| _ 60 | S:0.745 D:0.160 |
| _61 | R:0.944 |
| _ 62 | F:0.989 |
| _ 63 | S:0.889 |

(fortgesetzt)

| Position | Typ und Häufigkeit |
|---|---|
| _ 64 | G:0.992 |
| _ 65 | S:0.860 |
| _ 66 | G:0.882 |
| _ 67 | S:0.980 |
| _ 68 | G:0.968 |
| _ 69 | T:0.954 |
| _ 70 | D:0.782 E:0.180 |
| _ 71 | F:0.977 |
| _ 72 | T:0.898 |
| _ 73 | L:0.766 F:0.223 |
| _ 74 | T:0.930 |
| _ 75 | I:0.953 |
| _ 76 | S:0.870 |
| _ 77 | S:0.669 R:0.136 G:0.131 |
| _ 78 | L:0.907 |
| _ 79 | Q:0.753 E:0.164 |
| _ 80 | P:0.817 |
| _ 81 | E:0.803 D:0.177 |
| _ 82 | D:0.957 |
| _ 83 | F:0.656 V:0.143 I:0.140 |
| _84 | A:0.945 |
| _ 85 | T:0.598 V:0.283 |
| _ 86 | Y:0.989 |
| _ 87 | Y:0.919 |
| _ 88 | C:1.000 |
| _ 89 | Q:0.830 |
| _ 90 | Q:0.950 |
| _ 91 | Y:0.565 S:0.159 |
| _ 92 | Y:0.276 N:0.204 D:0.169 S:0.102 |
| _ 93 | S:0.327 T:0.199 N:0.193 |
| _ 94 | L:0.254 S:0.154 Y:0.150 F:0.121 T:0.116 |
| _ 95 | P:0.815 |
| _ 95a | -:0.908 |
| _95b | -:1.000 |
| _ 95c | -:1.000 |
| _95d | -:1.000 |
| _95e | -:1.000 |
| _95f | -:1.000 |
| _ 96 | Y:0.197 R:0.177 W:0.130 |
| _ 97 | T:0.897 |
| _ 98 | F:0.988 |
| _ 99 | G:1.000 |
| _100 | Q:0.612 G:0.275 |
| _101 | G:1.000 |
| _102 | T:0.953 |
| _103 | K:0.810 R:0.116 |
| _104 | V:0.714 L:0.240 |
| _105 | E:0.699 D:0.207 |
| _106 | I:0.717 |

(fortgesetzt)

| Position | Typ und Häufigkeit |
|----------|--------------------|
| _106a | -:1.000 |
| _107 | K:0.902 |
| _108 | R:0.959 |
| _109 | T:1.000 |

Tabelle 4

Variable Domänen der leichten Kette des Kappa-Typs der Maus

Ermittelte Konsensussequenz:

```
  1 DIVMTQSPASLSASLGERVTITCRASQSVSS------YLHWYQQKPGQSPKLLIYR
 51 ASNLASGVPDRFSGSGSGTDFTLTISSVEAEDLATYYCQQSNSYP------YTFGG
101 GTKLEI-KR
```

(SEQ ID NO:4 zeigt die Konsensussequenz ohne Einschübe)

| Position | Typ und Häufigkeit |
|----------|--------------------|
| _ 1 | D:0.707 E:0.106 Q:0.104 |
| _ 2 | I:0.813 V:0.121 |
| _ 3 | V:0.653 Q:0.223 |
| _ 4 | M:0.520 L:0.424 |
| _ 5 | T:0.878 |
| _ 6 | Q:0.995 |
| _ 7 | S:0.808 T:0.152 |
| _ 8 | P:0.871 |
| _ 9 | A:0.383 S:0.313 K:0.115 |
| _10 | S:0.571 I:0.177 |
| _ 11 | L:0.578 M:0.322 |
| _ 12 | S:0.476 A:0.257 P:0.117 |
| _ 13 | A:0.482 V:0.393 |
| _14 | S:0.874 |
| _ 15 | L:0.464 P:0.273 V:0.133 |
| _16 | G:0.977 |
| _ 17 | E:0.462 D:0.313 Q:0.188 |
| _ 18 | R:0.447 K:0.282 |
| _ 19 | V:0.693 A:0.192 I:0.103 |
| _ 20 | T:0.708 S:0.253 |
| _ 21 | I:0.607 M:0.224 L:0.122 |
| _ 22 | T:0.487 S:0.432 |
| _ 23 | C:0.984 |
| _ 24 | R:0.476 K:0.253 S:0.161 |
| _ 25 | A:0.812 S:0.166 |
| _ 26 | S:0.974 |

(fortgesetzt)

| Position | Typ und Häufigkeit |
|---|---|
| _ 27 | Q:0.524 S:0.231 E:0.133 |
| _ 28 | S:0.623 D:0.157 N:0.118 |
| _ 29 | V:0.411 I:0.383 L:0.176 |
| _ 30 | S:0.408 G:0.129 |
| _ 31 | S:0.227N:0.192T:0.158-:0.111 |
| _31a | -:0.569 S:0.256 |
| _31b | -:0.685 G:0.141 |
| _31c | -:0.685 G:0.102 |
| _31d | -:0.690 S:0.112 |
| _ 31e | -:0.821 T:0.103 |
| _ 31f: | -:0.924 |
| _ 32 | Y:0.652 N:0.122 |
| _ 33 | L:0.603 M:0.231 V:0.114 |
| _ 34 | H:0.330 A:0.227 N:0.155 |
| _ 35 | W:0.989 |
| _ 36 | Y:0.790 F:0.126 |
| _ 37 | Q:0.893 L:0.102 |
| _ 38 | Q:0.926 |
| _ 39 | K:0.879 |
| _ 40 | P:0.808 S:0.134 |
| _41 | G:0.773 |
| _ 42 | Q:0.450 K:0.151 G:0.105 |
| _ 43 | S:0.641 P:0.154 T:0.114 |
| _ 44 | P:0.888 |
| _ 45 | K:0.810 |
| _ 46 | L:0.802 |
| _ 47 | L:0.814 W:0.171 |
| _ 48 | I:0.938 |
| _ 49 | Y:0.880 |
| _ 50 | R:0.186 Y:0.164 S:0.147 G:0.137 A:0.102 |
| _ 51 | A:0.507 T:0.296 |
| _ 52 | S:0.893 |
| _ 53 | N:0.469 T:0.146 |
| _ 54 | L:0.630 R:0.262 |
| _ 55 | A:0.322 E:0.182 Y:0.123 |
| _ 56 | S:0.687 T:0.140 |
| _ 57 | G:0.997 |
| _ 58 | V:0.864 I:0.132 |
| _ 59 | P:0.961 |
| _ 60 | D:0.354 S:0.279 A:0.262 |
| _ 61 | R:0.976 |
| _ 62 | F:0.997 |
| _ 63 | S:0.776 T:0.192 |
| _ 64 | G:0.992 |
| _ 65 | S:0.981 |
| _ 66 | G:0.955 |
| _ 67 | S:0.969 |
| _ 68 | G:0.896 |
| _ 69 | T:0.862 |

(fortgesetzt)

| Position | Typ und Häufigkeit |
|----------|--------------------|
| _ 70 | D:0.675 S:0.226 |
| _ 71 | F:0.618 Y:0.373 |
| _ 72 | T:0.554 S:0.434 |
| _ 73 | L:0.901 |
| _ 74 | T:0.702 K:0.111 |
| _ 75 | I:0.977 |
| _ 76 | S:0.716 N:0.107 |
| _ 77 | S:0.567 P:0.142 R:0.127 |
| _ 78 | V:0.483 L:0.268 M:0.232 |
| _ 79 | E:0.667 Q:0.284 |
| _ 80 | A:0.479 S:0.124 P:0.115 E:0.106 |
| _ 81 | E:0.878 D:0.115 |
| _ 82 | D:0.976 |
| _ 83 | L:0.261 A:0.223 F:0.149 I:0.108 V:0.108 |
| _ 84 | A:0.764 G:0.170 |
| _ 85 | T:0.446 V:0.216 D:0.120 |
| _ 86 | Y:0.995 |
| _ 87 | Y:0.712 F:0.259 |
| _ 88 | C:0.999 |
| _ 89 | Q:0.669 L:0.131 |
| _ 90 | Q:0.905 |
| _ 91 | S:0.196 G:0.196 H:0.193 Y:0.117 W:0.100 |
| _ 92 | N:0.224 S:0.223 Y:0.169 |
| _ 93 | S:0.395 E:0.199 |
| _ 94 | Y:0.285 L:0.114 S:0.101 |
| _95 | P:0.938 |
| _ 95a | -:0.957 |
| _ 95b | -:0.990 |
| _ 95c | -:1.000 |
| _ 95d | -:1.000 |
| _ 95e | -:1.000 |
| _ 95f | -:1.000 |
| _ 96 | Y:0.263 L:0.255 W:0.172 R:0.114 |
| _ 97 | T:0.992 |
| _ 98 | F:1.000 |
| _ 99 | G:0.996 |
| _100 | G:0.631 A:0.244 S:0.114 |
| _101 | G:0.997 |
| _102 | T:1.000 |
| _103 | K:0.974 |
| _104 | L:0.995 |
| _105 | E:0.998 |
| _106 | I:0.763 |
| _106a | -:1.000 |
| _107 | K:0.958 |
| _108 | R:1.000 |

Tabelle 5

Variable Domänen der leichten Kette des Lambda-Typs des Menschen

Ermittelte Konsensussequenz:

```
  1  QSELTQPPS-VSVSPGQTVTISCSGDSLGIG------YVSWYQQKPGQAPKLVIYD
 51  DNKRPSGIPDRFSGSKSGNTASLTISGLQAEDEADYYCQSWDSSS------VVFGG
101  GTKLTVLGQP
```

(SEQ ID NO:5 zeigt die Konsensussequenz ohne Einschübe)

| Position | Typ und Häufigkeit |
|---|---|
| _ 1 | Q:0.557 S:0.211 |
| _ 2 | S:0.486 Y:0.392 |
| _ 3 | E:0.299 A:0.271 V:0.239 |
| _ 4 | L:0.995 |
| _ 5 | T:0.920 |
| _ 6 | Q:1.000 |
| _ 7 | P:0.865 |
| _ 8 | P:0.704 A:0.126 |
| _ 9 | S:0.911 |
| _ 10 | -:1.000 |
| _ 11 | V:0.858 |
| _ 12 | S:0.974 |
| _ 13 | V:0.410 G:0.345 A:0.129 |
| _ 14 | S:0.656 A:0.259 |
| _ 15 | P:0.826 L:0.123 |
| _ 16 | G:0.960 |
| _ 17 | Q:0.837 |
| _18 | T:0.544 S:0.291 R:0.111 |
| _19 | V:0.434 A:0.391 I:0.126 |
| _ 20 | T:0.518 R:0.259 |
| _21 | I:0.888 |
| _ 22 | S:0.518 T:0.457 |
| _23 | C:1.000 |
| _ 24 | S:0.471 T:0.243 |
| _ 25 | G:0.903 |
| _ 26 | D:0.389 S:0.214 T:0.183 |
| _ 27 | S:0.380 N:0.123 T:0.100 |
| _ 28 | L:0.366 S:0.322 |
| _ 29 | G:0.225 D:0.221 N:0.194 P:0.117 |
| _ 30 | I:0.264 V:0.230 K:0.102 |
| _ 31 | G:0.303 K:0.151 A:0.129 |
| _ 31a | -:0.449 S:0.133 G:0.114 D:0.113 |

(fortgesetzt)

| Position | Typ und Häufigkeit |
|---|---|
| _ 31b | -:0.486 N:0.168 Y:0.147 |
| _ 31c | -:0.682 N:0.166 |
| _ 31d | -:1.000 |
| _ 31e | -:1.000 |
| _ 31f | -:1.000 |
| _ 32 | Y:0.413 S:0.211 F:0.104 H:0.100 |
| _ 33 | V:0.647 A:0.228 |
| _ 34 | S:0.429 H:0.126 Y:0.110 |
| _ 35 | W:0.999 |
| _ 36 | Y:0.856 |
| _ 37 | Q:0.946 |
| _ 38 | Q:0.867 |
| _ 39 | K:0.275 R:0.229 H:0.215 L:0.132 |
| _40 | P:0.921 |
| _ 41 | G:0.846 |
| _ 42 | Q:0.453 K:0.224 T:0.156 |
| _ 43 | A:0.770 S:0.171 |
| _ 44 | P:0.999 |
| _ 45 | K:0.400 V:0.319 L:0.111 |
| _ 46 | L:0.777 |
| _ 47 | V:0.542 L:0.306 I:0,103 |
| _ 48 | I:0.822 V:0.131 |
| _ 49 | Y:0.824 F:0.123 |
| _ 50 | D:0.284 E:0.254 |
| _ 51 | D:0.338 V:0.194 N:0.173 |
| _ 52 | N:0.386 S:0.260 T:0.191 |
| _ 53 | K:0.255 Q:0.149 N:0.147 D:0.120 |
| _ 54 | R:0.950 |
| _ 55 | P:0.905 |
| _ 56 | S:0.875 |
| _ 57 | G:0.873 |
| _ 58 | I:0.595 V:0.369 |
| _ 59 | P:0.875 S:0.082 |
| _ 60 | D:0.392 E:0.326 L:0.109 |
| _61 | R:0.966 |
| _ 62 | F:0.967 |
| _ 63 | S:0.999 |
| _ 64 | G:0.913 |
| _ 65 | S:0.974 |
| _ 66 | K:0.437 S:0.193 N:0.190 |
| _ 67 | S:0.959 |
| _ 68 | G:0.859 |
| _ 69 | N:0.520 T:0.242 |
| _ 70 | T:0.565 S:0.275 |
| _71 | A:0.913 |
| _ 72 | S:0.491 T:0.436 |
| _ 73 | L:0.999 |
| _ 74 | T:0.812 A:0.116 |
| _ 75 | I:0.945 |

(fortgesetzt)

| Position | Typ und Häufigkeit |
|----------|--------------------|
| _ 76 | S:0.718 T:0.208 |
| _77 | G:0.828 R:0.120 |
| _78 | L:0.534 V:0.194 A:0.154 T:0.117 |
| _ 79 | Q:0.656 E:0.165 |
| _80 | A:0.460 S:0.175 T:0.171 V:0.127 |
| _ 81 | E:0.573 G:0.174 |
| _ 82 | D:0.971 |
| _83 | E:0.993 |
| _ 84 | A:0.974 |
| _ 85 | D:0.908 |
| _ 86 | Y:0.999 |
| _ 87 | Y:0.817 F:0.183 |
| _ 88 | C:0.999 |
| _ 89 | Q:0.473 S:0.203 |
| _ 90 | S:0.524 T:0.208 A:0.190 |
| _ 91 | W:0.438 Y:0.336 |
| _ 92 | D:0.590 |
| _ 93 | S:0.388 N:0.160 D:0.148 |
| _ 94 | S:0.537 G:0.156 |
| _ 95 | S:0.245 G:0.167 L:0.158 |
| _ 95a | -:0.343 S:0.156 N:0.103 |
| _ 95b | -:0.590 |
| _ 95c | -:0.941 |
| _ 95d | -:0.992 |
| _ 95e | -:1.000 |
| _ 95f | -:1.000 |
| _ 96 | V:0.344 P:0.101 |
| _ 97 | V:0.715 I:0.152 L:0.111 |
| _ 98 | F:0.999 |
| _ 99 | G:0.999 |
| _100 | G:0.808 |
| _101 | G:1.000 |
| _102 | T:0.999 |
| _103 | K:0.779 |
| _104 | L:0.669 V:0.330 |
| _105 | T:0.915 |
| _106 | V:0.999 |
| _106a | L:0.968 |
| _107 | G:0.728 R:0.205 |
| _108 | Q:0.993 |
| _109 | P:0.993 |

Tabelle 6

Variable Domänen der leichten Ketten des Lambda-Typs der Maus:

Ermittelte Konsensussequenz:

```
  1 QAVVTQESA-LTTSPGETVTLTCRSSTGAVTTSN---YANWVQEKPDHLFTGLIGG
 51 TNNRAPGVPARFSGSLIGDKAALTITGAQTEDEAIYFCALWYSNH------WVFGG
101 GTKLTVLGQP
```

(SEQ ID NO:6 zeig die Konsensussequenz ohne Einschübe)

| Position | Typ und Häufigkeit |
|----------|---------------------|
| 1 | Q:0.966 |
| 2 | A:0.999 |
| 3 | V:1.000 |
| 4 | V:0.999 |
| 5 | T:1.000 |
| 6 | Q:1.000 |
| 7 | E:0.894 Q:0.105 |
| 8 | S:1.000 |
| 9 | A:0.999 |
| 10 | -:1.000 |
| 11 | L:0.999 |
| 12 | T:0.999 |
| 13 | T:0.999 |
| 14 | S:1.000 |
| 15 | P:0.999 |
| 16 | G:0.994 |
| 17 | E:0.655 G:0.344 |
| 18 | T:0.999 |
| 19 | V:0.999 |
| 20 | T:0.646 I:0.353 |
| 21 | L:1.000 |
| 22 | T:0.990 |
| 23 | C:1.000 |
| 24 | R:0.999 |
| 25 | S:0.999 |
| 26 | S:0.800 T:0.175 |
| 27 | T:0.888 S:0.112 |
| 28 | G:0.999 |
| 29 | A:0.999 |
| 30 | V:0.991 |
| 31 | T:1.000 |
| 31a | T:0.989 |
| 31b | S:0.925 |

(fortgesetzt)

| Position | Typ und Häufigkeit |
|----------|--------------------|
| 31c | N:0.999 |
| 31d | -:1.000 |
| 31e | -:1.000 |
| 31f | -:1.000 |
| 32 | Y:0.999 |
| 33 | A:0.999 |
| 34 | N:0.990 |
| 35 | W:1.000 |
| 36 | V:0.919 |
| 37 | Q:1.000 |
| 38 | E:0.865 Q:0.135 |
| 39 | K:0.999 |
| 40 | P:0.990 |
| 41 | D:0.999 |
| 42 | H:0.999 |
| 43 | L:0.999 |
| 44 | F:0.999 |
| 45 | T:0.990 |
| 46 | G:0.999 |
| 47 | L:0.990 |
| 48 | I:0.982 |
| 49 | G:0.999 |
| 50 | G:0.984 |
| 51 | T:0.992 |
| 52 | N:0.609 S:0.343 |
| 53 | N:0.844 |
| 54 | R:0.999 |
| 55 | A:0.859 T:0.105 |
| 56 | P:0.999 |
| 57 | G:1.000 |
| 58 | V:0.999 |
| 59 | P:1.000 |
| 60 | A:0.632 V:0.367 |
| 61 | R:1.000 |
| 62 | F:1.000 |
| 63 | S:1.000 |
| 64 | G:1.000 |
| 65 | S:1.000 |
| 66 | L:0.999 |
| 67 | I:0.999 |
| 68 | G:1.000 |
| 69 | D:0.888 N:0.110 |
| 70 | K:0.999 |
| 71 | A:0.999 |
| 72 | A:0.999 |
| 73 | L:1.000 |
| 74 | T:0.999 |
| 75 | I:1.000 |
| 76 | T:0.999 |

(fortgesetzt)

| Position | Typ und Häufigkeit |
|---|---|
| 77 | G:0.999 |
| 78 | A:0.928 |
| 79 | Q:1.000 |
| 80 | T:0.999 |
| 81 | E:1.000 |
| 82 | D:1.000 |
| 83 | E:0.657 D:0.343 |
| 84 | A:1.000 |
| 85 | I:0.615 M:0.385 |
| 86 | Y:1.000 |
| 87 | F:0.999 |
| 88 | C:1.000 |
| 89 | A:0.992 |
| 90 | L:0.999 |
| 91 | W:0.999 |
| 92 | Y:0.897 |
| 93 | S:0.895 |
| 94 | N:0.763 T:0.236 |
| 95 | H:0.929 |
| 95a | -:0.976 |
| 95b | -:0.999 |
| 95c | -:0.999 |
| 95d | -:0.999 |
| 95e | -:1.000 |
| 95f | -:1.000 |
| 96 | W:0.510 F:0.327 Y:0.107 |
| 97 | V:0.767 I:0.176 |
| 98 | F:1.000 |
| 99 | G:0.936 |
| 100 | G:0.841 S:0.159 |
| 101 | G:1.000 |
| 102 | T:0.996 |
| 103 | K:1.000 |
| 104 | L:0.549 V:0.451 |
| 105 | T:1.000 |
| 106 | V:1.000 |
| 106a | L:1.000 |
| 107 | G:1.000 |
| 108 | Q:0.874 X:0.126 |
| 109 | P:1.000 |

**Patentansprüche**

1. Verfahren zur Herstellung eines stabilitätsverbesserten funktionellen Antikörpers, funktionellen Derivats oder Fragments davon in einem eukaryontischen oder prokaryontischen Mikroorganismus durch Transformation des Organismus mit einem Expressionsvektor, der ein rekombinantes Gen enthält, welches für den genannten Antikörper, das Derivat oder Fragment codiert, **dadurch gekennzeichnet, daß**

a) die Aminosäuresequenz mindestens einer der variablen Domänen des Antikörpers mit den Sequenzen SEQ ID NO:1-6, erwähnt in Tabellen 1 - 6 der Beschreibung, verglichen und die Tabelle ausgewählt wird, deren Konsensussequenz die höchste Homologie zu dieser Domäne zeigt,

b) im Gen dieser variablen Domäne, mindestens ein Codon einer Aminosäure ersetzt wird, und zwar

ba) falls diese Aminosäure nicht in ihrer Position in dieser ausgewählten Tabelle genannt ist, durch ein Codon für eine der genannten Aminosäuren und/oder

bb) falls diese Aminosäure in ihrer Position in der ausgewählten Tabelle genannt ist, durch ein Codon für eine der genannten Aminosäuren mit einer größeren Häufigkeit

c) und der prokaryontische oder eukaryontische Mikroorganismus mit dem so modifizierten Gen transformiert und der Antikörper, das Fragment oder Derivat mit der gewünschten Aktivität exprimiert wird.

2. Verfahren nach Anspruch 1, **dadurch gekennzeichnet, daß** ein modifiziertes Gen verwendet wird, in dem mindestens ein Codon für eine Aminosäure ersetzt ist, und zwar

a) im Gen der variablen Domäne der schweren Kette des Menschen,

aa) falls diese Aminosäure nicht in ihrer Position in Tabelle 1 genannt ist, durch ein Codon für eine der genannten Aminosäuren und/oder

ab) falls diese Aminosäure in ihrer Position in Tabelle 1 genannt ist, durch ein Codon für eine der genannten Aminosäure mit einer größeren Häufigkeit,

b) im Gen der variablen Domäne der schweren Kette der Maus

ba) falls diese Aminosäure nicht in ihrer Position in Tabelle 2 genannt ist, durch ein Codon für eine der genannten Aminosäuren und/oder

bb) falls die Aminosäure in ihrer Position in Tabelle 2 genannt ist, durch ein Codon für eine der genannten Aminosäuren mit einer größeren Häufigkeit,

c) im Gen der variablen Domäne der leichten Kette des Kappa-Typs des Menschen

ca) falls diese Aminosäure nicht in ihrer Position in Tabelle 3 genannt ist, durch ein Codon für eine der genannten Aminosäuren und/oder

cb) falls die Aminosäure in ihrer Position in Tabelle 3 genannt ist, durch ein Codon für eine der genannten Aminosäuren mit einer größeren Häufigkeit,

d) im Gen der variablen Domäne der leichten Kette des Kappa-Typs der Maus

da) falls diese Aminosäure nicht in ihrer Position in Tabelle 4 genannt ist, durch ein Codon für eine der genannten Aminosäuren und/oder

db) falls die Aminosäure in ihrer Position in Tabelle 4 genannt ist, durch ein Codon für eine der genannten Aminosäuren mit einer größeren Häufigkeit,

e) im Gen der variablen Domäne der leichten Kette des λ-Typs des Menschen

ea) falls diese Aminosäure nicht in ihrer Position in Tabelle 5 genannt ist, durch ein Codon für eine der genannten Aminosäuren und/oder

eb) falls die Aminosäure in ihrer Position in Tabelle 5 genannt ist, durch ein Codon für eine der genannten Aminosäuren mit einer größeren Häufigkeit

f) im Gen der variablen Domäne der leichten Kette des λ-Typs der Maus

fa) falls diese Aminosäure nicht in ihrer Position in Tabelle 6 genannt ist, durch ein Codon für eine der genannten Aminosäuren und/oder

fb) falls die Aminosäure in ihrer Position in Tabelle 6 genannt ist, durch ein Codon für eine der genannten Aminosäuren mit einer größeren Häufigkeit.

3. Verfahren nach den Ansprüchen 1 oder 2, **dadurch gekennzeichnet, daß** der Antikörper, das Fragment oder Derivat aus dem Organismus isoliert und gereinigt wird.

4. Verfahren nach den Ansprüchen 1 - 3, **dadurch gekennzeichnet, daß** die Expression in einem prokaryontischen oder eukaryontischen Mikroorganismus erfolgt und der Antikörper, das Fragment oder Derivat im Cytosol funktionell entsteht.

5. Verfahren nach den Ansprüchen 1 - 4, **dadurch gekennzeichnet, daß** mindestens zwei Codons ausgetauscht werden.

6. Verfahren nach den Ansprüchen 1 - 5, **dadurch gekennzeichnet, daß** zusätzlich mindestens eines der disulfidbrückenbildenden Cysteine im Antikörper, Fragment oder Derivat durch eine andere Aminosäure ersetzt ist.

7. Verfahren nach Anspruch 6, **dadurch gekennzeichnet, daß** alle disulfidbrückenbildenden Cysteine ersetzt sind.

8. Hybridprotein aus einer $V_H$- und $V_L$-Domäne eines Antikörpers, welches über eine intermolekulare Cystinbrücke verknüpft ist, und bei dem die Cysteine an den Positionen 22 und/oder 99 (schwere Kette), 23 und/oder 88 (leichte Kette) durch andere Aminosäuren ersetzt sind und mindestens eine weitere Aminosäure, vorzugsweise außerhalb des CDR-Bereichs, gemäß dem Verfahren der Ansprüche 1 - 5, ersetzt ist.

9. Verfahren nach dem Anspruch 3, daß der Antikörper, das Fragment oder Derivat unter reduzierenden Bedingungen in vitro zum funktionellen Protein gefaltet wird.

**Claims**

1. Process for the production of a functional antibody having improved stability or of a functional derivative or fragment thereof in a eukaryotic or prokaryotic microorganism by transforming the organism with an expression vector which contains a recombinant gene which codes for the said antibody, the derivative or fragment, wherein

a) the amino acid sequence of at least one of the variable domains of the antibody is compared with the sequences SEQ ID NO: 1-6 mentioned in tables 1 - 6 of the description and the table is selected whose consensus sequence has the highest homology to this domain,

b) at least one codon of an amino acid is replaced in the gene of this variable domain and namely

ba) by a codon for one of the said amino acids if this amino acid is not mentioned in its position in this selected table and/or

bb) by a codon for one of the said amino acids with a higher abundance if this amino acid is mentioned in its position in the selected table

c) and the prokaryotic or eukaryotic microorganism is transformed with the gene modified in this manner and the antibody, the fragment or derivative having the desired activity is expressed.

2. Process as claimed in claim 1, wherein a modified gene is used in which at least one codon for an amino acid is replaced and namely

a) in the gene of the variable domain of the human heavy chain,

aa) by a codon for one of the said amino acids if this amino acid is not mentioned in its position in table 1 and/or

ab) by a codon for one of the said amino acids with a higher abundance if this amino acid is mentioned in its position in table 1,

b) in the gene of the variable domain of the heavy chain of the mouse

ba) by a codon for one of the said amino acids if this amino acid is not mentioned in its position in table 2 and/or

bb) by a codon for one of the said amino acids with a higher abundance if this amino acid is mentioned in its position in table 2,

c) in the gene of the variable domain of the human light chain of the kappa type

ca) by a codon for one of the said amino acids if this amino acid is not mentioned in its position in table 3 and/or

cb) by a codon for one of the said amino acids with a higher abundance if this amino acid is mentioned in its position in table 3,

d) in the gene of the variable domain of the light chain of the kappa type of the mouse

da) by a codon for one of the said amino acids if this amino acid is not mentioned in its position in table 4 and/or

db) by a codon for one of the said amino acids with a higher abundance if this amino acid is mentioned in its position in table 4,

e) in the gene of the variable domain of the human light chain of the λ type

ea) by a codon for one of the said amino acids if this amino acid is not mentioned in its position in table 5 and/or

eb) by a codon for one of the said amino acids with a higher abundance if this amino acid is mentioned in its position in table 5,

f) in the gene of the variable domain of the light chain of the λ type of the mouse

fa) by a codon for one of the said amino acids if this amino acid is not mentioned in its position in table 6 and/or

fb) by a codon for one of the said amino acids with a higher abundance if this amino acid is mentioned in its position in table 6.

3. Process as claimed in one of the claims 1 or 2, wherein the antibody, the fragment or derivative is isolated from the organism and purified.

4. Process as claimed in one of the claims 1 - 3, wherein the expression takes place in a prokaryotic or eukaryotic microorganism and the antibody, the fragment or derivative is formed in the cytosol in a functional form.

5. Process as claimed in one of the claims 1 - 4, wherein at least two codons are exchanged.

6. Process as claimed in one of the claims 1 - 5, wherein additionally at least one of the cysteins forming disulfide bridges in the antibody, fragment or derivative is replaced by another amino acid.

7. Process as claimed in claim 6, wherein all cysteines forming disulfide bridges are replaced.

**8.** Hybrid protein comprising a $V_H$ and $V_L$ domain of an antibody which is linked by an intermolecular cystine bridge and in which the cysteines at positions 22 and/or 99 (heavy chain), 23 and/or 88 (light chain) are replaced by other amino acids and at least one other amino acid, preferably outside the CDR region, is replaced according to the process as claimed in claims 1 - 5.

**9.** Process as claimed in claim 3, wherein the antibody, the fragment or derivative are folded in vitro under reducing conditions to form the functional protein.

**Revendications**

**1.** Procédé de préparation d'un anticorps fonctionnel à stabilité améliorée, d'un dérivé ou d'un fragment fonctionnel de celui-ci, dans un micro-organisme eucaryotique ou procaryotique, par transformation de l'organisme avec un vecteur d'expression qui contient un gène recombiné codant l'anticorps, le dérivé ou fragment mentionné, **caractérisé en ce que**

a) la séquence des acides aminés d'au moins un des domaines variables de l'anticorps est comparée avec les séquences SEQ ID NO:1-6, mentionnées dans les tableaux 1 - 6 de la description, et que le tableau soit choisi dont la séquence consensuelle montre la plus forte homologie avec ce domaine,
b) dans le gène de ce domaine variable, au moins un codon d'un acide aminé est remplacé, de manière à ce que

ba) si cet acide aminé n'est pas nommé dans sa position dans le tableau choisi, par un codon pour un des acides aminés nommés et/ou
bb) si cet acide aminé est nommé dans sa position dans le tableau choisi, par un codon pour un des acides aminés nommés avec une plus grande fréquence

c) et le micro-organisme procaryotique ou eucaryotique est transformé avec le gène ainsi modifié et l'anticorps, le fragment ou le dérivé ayant l'activité souhaité est exprimé.

**2.** Procédé selon la revendication 1, **caractérisé en ce que** un gène modifié est utilisé, dans lequel au moins un codon pour un acide aminé est remplacé, pour préciser

a) dans le gène du domaine variable de la chaîne lourde chez l'homme,

aa) si cet acide aminé n'est pas nommé dans sa position dans le tableau 1, par un codon pour un des acides aminés nommés et/ou
ab) si cet acide aminé est nommé dans sa position dans le tableau 1, par un codon pour un des acides aminés nommés avec une plus grande fréquence,

b) dans le gène du domaine variable de la chaîne lourde chez la souris,

ba) si cet acide aminé n'est pas nommé dans sa position dans le tableau 2, par un codon pour un des acides aminés nommés et/ou
bb) si cet acide aminé est nommé dans sa position dans le tableau 2, par un codon pour un des acides aminés nommés avec une plus grande fréquence,

c) dans le gène du domaine variable de la chaîne légère du type kappa chez l'homme

ca) si cet acide aminé n'est pas nommé dans sa position dans le tableau 3, par un codon pour un des acides aminés nommés et/ou
cb) si cet acide aminé est nommé dans sa position dans le tableau 3, par un codon pour un des acides aminés nommés avec une plus grande fréquence,

d) dans le gène du domaine variable de la chaîne légère de type kappa chez la souris

da) si cet acide aminé n'est pas nommé dans sa position dans le tableau 4, par un codon pour un des acides aminés nommés et/ou
db) si l'acide aminé est nommé dans sa position dans le tableau 4, par un codon pour un des acides aminés nommés avec une plus grande fréquence,

e) dans le gène du domaine variable de la chaîne légère de type λ chez l'homme

ea) si cet acide aminé n'est pas nommé dans sa position dans le tableau 5, par un codon pour un des acides aminés nommés et/ou
eb) si cet acide aminé est nommé dans sa position dans le tableau 5, par un codon pour un des acides aminés nommés avec une plus grande fréquence,

f) dans le gène du domaine variable de la chaîne légère du type λ de la souris

fa) si cet acide aminé n'est pas nommé dans sa position dans le tableau 6, par un codon pour un des acides aminés nommés et/ou
fb) si l'acide aminé est nommé dans sa position dans le tableau 6, par un codon pour un des acides aminés nommés avec une plus grande fréquence.

3. Procédé selon les revendications 1 ou 2, **caractérisé en ce que** l'anticorps, le fragment ou le dérivé est isolé à partir de l'organisme et purifié.

4. Procédé selon les revendications 1 - 3, **caractérisé en ce que** l'expression a lieu dans un micro-organisme procaryotique ou eucaryotique et que l'anticorps, le fragment ou le dérivé naît d'une manière fonctionnelle du cytosol.

5. Procédé selon les revendications 1 - 4, **caractérisé en ce qu'**au moins deux codons sont échangés.

6. Procédé selon les revendications 1 - 5, **caractérisé en ce que** dans l'anticorps, le fragment ou le dérivé au moins une des cystéines formant des ponts disulfure est remplacée par un autre acide aminé

7. Procédé selon la revendication 6, **caractérisé en ce que** toutes les cystéines formant des ponts disulfure sont remplacées.

8. Protéine hybride, composée d'un domaine $V_H$ et $V_L$ d'un anticorps, qui est relié par un pont cystéine intermoléculaire, et chez laquelle les cystéines des positions 22 et/ou 99 (chaîne lourde), 23 et/ou 88 (chaîne légère) sont remplacées par d'autres acides aminés et ou au moins un autre acide aminé, de préférence à l'extérieur de la région CDR, est remplacé conformément au procédé selon les revendications 1- 5.

9. Procédé selon la revendication 3, dans lequel l'anticorps, le fragment ou le dérivé est replié in vitro pour former une protéine fonctionnelle, dans des conditions réductrices.

**Fig. 1**

# Fig. 2

# Fig. 3